(19) Europäisches Patentamt
European Patent Office
Office européen des brevets

(11) **EP 3 760 053 A1**

(12) **EUROPEAN PATENT APPLICATION**
published in accordance with Art. 153(4) EPC

(43) Date of publication:
**06.01.2021 Bulletin 2021/01**

(21) Application number: **19764681.3**

(22) Date of filing: **09.03.2019**

(51) Int Cl.:
*A23L 29/10* [(2016.01)]  *A23L 33/105* [(2016.01)]
*A61K 9/107* [(2006.01)]  *A61K 9/48* [(2006.01)]
*A61K 31/01* [(2006.01)]  *A61K 31/015* [(2006.01)]
*A61K 31/045* [(2006.01)]  *A61K 31/047* [(2006.01)]
*A61K 31/122* [(2006.01)]  *A61K 36/05* [(2006.01)]
*A61K 47/02* [(2006.01)]  *A61K 47/10* [(2017.01)]
*A61K 47/14* [(2017.01)]  *A61K 47/26* [(2006.01)]
*A61P 17/18* [(2006.01)]  *A61P 27/02* [(2006.01)]
*A61P 29/00* [(2006.01)]  *A61P 35/00* [(2006.01)]
*A61P 39/06* [(2006.01)]

(86) International application number:
**PCT/JP2019/009549**

(87) International publication number:
**WO 2019/172454 (12.09.2019 Gazette 2019/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.03.2018 JP 2018043661**

(71) Applicant: **AstaReal Co., Ltd.**
**Nakaniikawa-gun, Toyama 930-0397 (JP)**

(72) Inventors:
• **SHITAKA Kazuna**
**Nakaniikawa-gun, Toyama 930-0397 (JP)**
• **SASAKI Ryoji**
**Nakaniikawa-gun, Toyama 930-0397 (JP)**
• **SAKAGUCHI Rina**
**Nakaniikawa-gun, Toyama 930-0397 (JP)**
• **TOMINAGA Kumi**
**Nakaniikawa-gun, Toyama 930-0397 (JP)**

(74) Representative: **Prüfer & Partner mbB**
**Patentanwälte · Rechtsanwälte**
**Sohnckestraße 12**
**81479 München (DE)**

(54) **EMULSION COMPOSITION**

(57) Provided is an emulsion composition that is capable of enhancing systemic absorption for various carotenoids, well compatible with soft capsules, and capable of being formulated into a soft capsule formulation, and that homogeneously disperses raw materials including carotenoids and emulsifiers in the soft capsule formulation. An emulsion composition containing a carotenoid, and two or more specific emulsifiers selected from the group consisting of tetraglycerin monooleate, decaglycerin monolaurate, sucrose stearate, and sucrose laurate.

Fig. 2

| | Example 1 | Example 2 | Example 3 | Example 4 | Example 5 | Example 6 | Example 7 | Example 8 |
|---|---|---|---|---|---|---|---|---|
| AUC ratio for astaxanthin | 2.7 | 1.5 | 2.4 | 1.7 | 1.5 | 1.3 | 1.9 | 1.2 |
| AUC ratio for lutein | — | 1.4 | 1.7 | 1.3 | 1.6 | 1.2 | 1.3 | 1.3 |
| AUC ratio for zeaxanthin | — | 2.5 | 10.1 | 9.6 | 10.8 | 2.8 | 4.1 | 3.3 |

| | Example 9 | Example 10 | Example 11 | Example 12 | Example 13 | Example 14 | Example 15 | Example 16 |
|---|---|---|---|---|---|---|---|---|
| AUC ratio for astaxanthin | 1.9 | 1.2 | 2.3 | 1.1 | 1.2 | 2.0 | 1.3 | 1.8 |
| AUC ratio for lutein | 1.4 | 1.2 | — | 1.0 | 1.1 | 1.3 | 1.1 | — |
| AUC ratio for zeaxanthin | 4.7 | 3.7 | — | 3.6 | 5.4 | 3.6 | 4.9 | — |

| | Example 17 | Example 18 | Example 19 | Example 20 | Example 21 | Example 22 | Example 23 | Example 24 |
|---|---|---|---|---|---|---|---|---|
| AUC ratio for astaxanthin | 1.2 | 1.1 | 1.5 | 1.6 | 1.9 | 1.4 | 2.1 | 1.7 |
| AUC ratio for lutein | 1.4 | 1.5 | 1.0 | 1.1 | 1.4 | 1.3 | 1.7 | 1.3 |
| AUC ratio for zeaxanthin | 1.4 | 1.9 | 0.6 | 2.0 | 4.0 | 3.4 | 3.7 | 5.0 |

| | Example 25 | Example 26 | Example 27 | Example 28 | Example 29 | Example 30 | Example 31 | Example 32 |
|---|---|---|---|---|---|---|---|---|
| AUC ratio for astaxanthin | 1.3 | 1.2 | 1.6 | 2.1 | 1.8 | 1.4 | 2.2 | 2.1 |
| AUC ratio for lutein | 1.3 | 0.9 | 1.2 | 1.2 | 1.1 | 1.0 | 1.2 | 1.2 |
| AUC ratio for zeaxanthin | 5.5 | 2.6 | 2.5 | 1.4 | 0.8 | 0.8 | 1.9 | 1.5 |

| | Example 33 | Example 34 | Example 35 |
|---|---|---|---|
| AUC ratio for astaxanthin | 1.8 | 4.6 | 2.0 |
| AUC ratio for lutein | — | — | — |
| AUC ratio for zeaxanthin | — | — | — |

| | Example 36 | Example 37 | Example 38 | Example 39 | Example 40 | Example 41 | Example 42 |
|---|---|---|---|---|---|---|---|
| AUC ratio for astaxanthin | 1.5 | 2.6 | 1.8 | 2.6 | 2.2 | — | 1.8 |
| AUC ratio for lutein | — | — | — | — | — | 1.3 | 1.3 |
| AUC ratio for zeaxanthin | — | — | — | — | — | 13.3 | 1.8 |

EP 3 760 053 A1

**Description**

Technical Field

The present invention relates to an emulsion composition, specifically, an emulsion composition containing a carotenoid, and two or more specific emulsifiers selected from the group consisting of tetraglycerin monooleate, decaglycerin monolaurate (decaglyceryl monolaurate), sucrose stearate, and sucrose laurate.

Background Art

Most of poorly-soluble bioactive components handled in the field of health foods, drugs, and quasi drugs suffer from problems with systemic absorption. Carotenoids, which have recently been attracting attention for the high functionality, are also poorly-soluble bioactive components suffering from such problems with systemic absorption, and typically handled in the form of an emulsion composition to enhance the systemic absorption. Emulsion compositions are classified into those of emulsified type formed by adding water and an emulsifier to an oil component, and those of self-emulsifying and pre-emulsified types formed by adding an emulsifier to an oil component. Emulsion compositions of emulsified type generally have a characteristic of quick dissolution in water, and hence are used for beverages, cosmetics, and so on. Emulsion compositions of self-emulsifying and pre-emulsified types are used for soft capsule formulations, in which the blend ratio of water or glycerin is limited.

Soft capsule formulations are formulations formed by enclosing a drug, a supplement, or the like in powder, liquid, or in another form in a capsule shell and molded, and this dosage form is suitable for oral administration of bioactive components being poorly-soluble, those having distinct odor or bitter taste, and those being instable to oxygen or light. While such a component is needed to be homogenously dispersed in a soft capsule formulation, occasionally a raw material containing the component is unevenly distributed in formulating into a soft capsule formulation. In particular, when a powdery substance such as sucrose fatty acid ester is used as a raw material, the raw material tends to be dispersed in an inhomogeneous manner. On the other hands, carotenoids are capable of being formulated into a soft capsule formulation if being in an emulsion composition of self-emulsifying or pre-emulsified type, but the systemic absorption is low. Therefore, development of an emulsion composition of emulsified type that is capable of being formulated into a soft capsule formulation with high systemic absorption is demanded.

Emulsion compositions that enhance systemic absorption for carotenoids and are capable of being formulated into a soft capsule formulation were previously developed, and examples thereof include a fat-reducing agent containing, as an active ingredient, a carotenoid-containing composition containing a crystalline carotenoid with 90% by mass thereof being amorphous, and a (poly)glycerin fatty acid ester with the number of glycerin units being 1 to 6 and the number of fatty acid units being 1 to 6, wherein the (poly)glycerin fatty acid ester has at least one hydroxy group in a glycerin unit (Patent Literature 1).

Citation List

Patent Literature

Patent Literature 1: Japanese Patent Laid-Open No. 2012-206972

Summary of Invention

Technical Problem

However, the emulsion composition in Patent Literature 1 is an emulsion composition that enhances systemic absorption only for lycopene, one of carotenoids, and is capable of being formulated into a soft capsule formulation, never an emulsion composition that is capable of enhancing systemic absorption for various carotenoids, and capable of being formulated into a soft capsule formulation, thus having versatility for carotenoids.

The present invention was made to solve the above-described problems, and an object of the present invention is to provide an emulsion composition that is capable of enhancing systemic absorption for various carotenoids, well compatible with soft capsules, and capable of being formulated into a soft capsule formulation, and that homogeneously disperses raw materials including carotenoids and emulsifiers in the soft capsule formulation.

Solution to Problem

The present inventors diligently studied to find that an emulsion composition containing a carotenoid, and two or more specific emulsifiers selected from the group consisting of tetraglycerin monooleate, decaglycerin monolaurate, sucrose stearate, and sucrose laurate is capable of enhancing systemic absorption for various carotenoids, well compatible with soft capsules, and capable of being formulated into a soft capsule formulation, and homogeneously disperses raw materials including carotenoids and emulsifiers in the soft capsule formulation, thus completing the following invention.

(1) An emulsion composition containing a carotenoid and an emulsifier, the emulsifier being any combination of the following (a), (b), (c), (d), (e), (f), (g), (h), (i), and (j):

> (a) tetraglycerin monooleate and decaglycerin monolaurate;
> (b) tetraglycerin monooleate and sucrose stearate;
> (c) tetraglycerin monooleate and sucrose laurate;
> (d) decaglycerin monolaurate and sucrose laurate;
> (e) sucrose stearate and sucrose laurate;
> (f) tetraglycerin monooleate, decaglycerin monolaurate, and sucrose stearate;
> (g) tetraglycerin monooleate, decaglycerin monolaurate, and sucrose laurate;
> (h) tetraglycerin monooleate, sucrose stearate, and sucrose laurate;
> (i) decaglycerin monolaurate, sucrose stearate, and sucrose laurate; and
> (j) tetraglycerin monooleate, decaglycerin monolaurate, sucrose stearate, and sucrose laurate.

(2) An emulsion composition containing a carotenoid, and decaglycerin monolaurate and sucrose stearate, and being free of lecithin.

(3) The emulsion composition according to (1) or (2), containing an alcohol; here, examples of the alcohol can include, but are not limited to, monohydric alcohols such as methanol and ethanol, dihydric alcohols such as propylene glycol, and (polyhydric) sugar alcohols such as xylitol, sorbitol, lactitol, and erythritol.

(4) The emulsion composition according to any one of (1) to (3), wherein the carotenoid is one or more selected from the group consisting of lutein, zeaxanthin, astaxanthin, lycopene, β-carotene, γ-carotene, phytofluene, phytoene, canthaxanthin, β-cryptoxanthin, capsanthin, fucoxanthin, and fatty acid esters thereof .

(5) The emulsion composition according to (4), wherein the astaxanthin is derived from a Haematococcus algae extract.

(6) The emulsion composition according to any one of (1) to (5), containing a mineral and/or a vitamin.

(7) The emulsion composition according to any one of (1) to (6), having a self-emulsifying property.

(8) A soft capsule formulation containing the emulsion composition according to any one of (1) to (7).

(9) A method for producing the emulsion composition according to (1), the method including the steps of:

> preparing an aqueous phase by mixing and dissolving at least sucrose stearate and/or sucrose laurate in water;
> preparing an oil phase by mixing and dissolving at least a carotenoid, and tetraglycerin monooleate and/or decaglycerin monolaurate; and
> subsequently mixing the aqueous phase and the oil phase together to obtain an emulsion composition.

(10) A method for producing the emulsion composition according to (1), the method including the steps of:

> preparing an aqueous phase by mixing and dissolving at least sucrose stearate in water;
> preparing an oil phase by mixing and dissolving at least a carotenoid, and one or more fatty acid esters selected from the group consisting of sucrose laurate, tetraglycerin monooleate, and decaglycerin monolaurate; and
> subsequently mixing the aqueous phase and the oil phase together to obtain an emulsion composition.

(11) A method for producing the emulsion composition according to (1), the method including the steps of:

> preparing an aqueous phase by mixing and dissolving at least sucrose laurate in water;
> preparing an oil phase by mixing and dissolving at least a carotenoid, and one or more fatty acid esters selected from the group consisting of sucrose stearate, tetraglycerin monooleate, and decaglycerin monolaurate; and
> subsequently mixing the aqueous phase and the oil phase together to obtain an emulsion composition.

(12) A method for producing the emulsion composition according to (1), the method including the steps of:

> preparing an aqueous phase by mixing and dissolving at least sucrose stearate and sucrose laurate in water;
> preparing an oil phase by mixing and dissolving at least a carotenoid and an oil or fat; and

subsequently mixing the aqueous phase and the oil phase together to obtain an emulsion composition.

(13) A method for producing the emulsion composition according to (1), the method including the steps of:

preparing an aqueous phase by mixing and dissolving a water-soluble substance, as necessary, in water;
preparing an oil phase by mixing and dissolving at least a carotenoid, and two or more fatty acid esters selected from the group consisting of sucrose stearate, sucrose laurate, tetraglycerin monooleate, and decaglycerin monolaurate; and
subsequently mixing the aqueous phase and the oil phase together to obtain an emulsion composition.

(14) A method for producing the emulsion composition according to (2), the method including the steps of:

preparing an aqueous phase by mixing and dissolving at least sucrose stearate in water;
preparing an oil phase by mixing and dissolving at least a carotenoid and decaglycerin monolaurate, without mixing and dissolving lecithin; and
subsequently mixing the aqueous phase and the oil phase together to obtain an emulsion composition.

(15) A method for producing the emulsion composition according to (2), the method including the steps of:

preparing an aqueous phase by mixing and dissolving a water-soluble substance, as necessary, in water;
preparing an oil phase by mixing and dissolving at least a carotenoid, sucrose stearate, and decaglycerin monolaurate, without mixing and dissolving lecithin; and
subsequently mixing the aqueous phase and the oil phase together to obtain an emulsion composition.

(16) The method for producing an emulsion composition according to any one of (9) to (15), the method including the step of adding and mixing an alcohol in at least any step of the step of preparing the aqueous phase, the step of preparing the oil phase, the step of mixing the aqueous phase and the oil phase together, and the step of mixing the water and the oil phase together.

(17) A method for producing a soft capsule formulation, the method including the steps of:

preparing an aqueous phase by mixing and dissolving at least sucrose stearate and/or sucrose laurate in water;
preparing an oil phase by mixing and dissolving at least a carotenoid, and tetraglycerin monooleate and/or decaglycerin monolaurate;
subsequently mixing the aqueous phase and the oil phase together to obtain the emulsion composition according to (1); and
subsequently enclosing a content containing the emulsion composition in a soft capsule shell.

(18) A method for producing a soft capsule formulation, the method including the steps of:

preparing an aqueous phase by mixing and dissolving at least sucrose stearate in water;
preparing an oil phase by mixing and dissolving at least a carotenoid, and one or more fatty acid esters selected from the group consisting of sucrose laurate, tetraglycerin monooleate, and decaglycerin monolaurate;
subsequently mixing the aqueous phase and the oil phase together to obtain the emulsion composition according to (1); and
subsequently enclosing a content containing the emulsion composition in a soft capsule shell.

(19) A method for producing a soft capsule formulation, the method including the steps of:

preparing an aqueous phase by mixing and dissolving at least sucrose laurate in water;
preparing an oil phase by mixing and dissolving at least a carotenoid, and one or more fatty acid esters selected from the group consisting of sucrose stearate, tetraglycerin monooleate, and decaglycerin monolaurate;
subsequently mixing the aqueous phase and the oil phase together to obtain the emulsion composition according to (1); and
subsequently enclosing a content containing the emulsion composition in a soft capsule shell.

(20) A method for producing a soft capsule formulation, the method including the steps of:

preparing an aqueous phase by mixing and dissolving at least sucrose stearate and sucrose laurate in water;

preparing an oil phase by mixing and dissolving at least a carotenoid and an oil or fat;
subsequently mixing the aqueous phase and the oil phase together to obtain the emulsion composition according to (1); and
subsequently enclosing a content containing the emulsion composition in a soft capsule shell.

(21) A method for producing a soft capsule formulation, the method including the steps of:

preparing an aqueous phase by mixing and dissolving a water-soluble substance, as necessary, in water;
preparing an oil phase by mixing and dissolving at least a carotenoid, and two or more fatty acid esters selected from the group consisting of sucrose stearate, sucrose laurate, tetraglycerin monooleate, and decaglycerin monolaurate;
subsequently mixing the aqueous phase and the oil phase together to obtain the emulsion composition according to (1); and
subsequently enclosing a content containing the emulsion composition in a soft capsule shell.

(22) A method for producing a soft capsule formulation, the method including the steps of:

preparing an aqueous phase by mixing and dissolving at least sucrose stearate in water;
preparing an oil phase by mixing and dissolving at least a carotenoid and decaglycerin monolaurate, without mixing and dissolving lecithin;
subsequently mixing the aqueous phase and the oil phase together to obtain the emulsion composition according to (2); and
subsequently enclosing a content containing the emulsion composition in a soft capsule shell.

(23) A method for producing a soft capsule formulation, the method including the steps of:

preparing an aqueous phase by mixing and dissolving a water-soluble substance, as necessary, in water;
preparing an oil phase by mixing and dissolving at least a carotenoid, sucrose stearate, and decaglycerin monolaurate, without mixing and dissolving lecithin;
subsequently mixing the aqueous phase and the oil phase together to obtain the emulsion composition according to (2); and
subsequently enclosing a content containing the emulsion composition in a soft capsule shell.

(24) The method for producing a soft capsule formulation according to any one of (17) to (23), the method including the step of adding and mixing an alcohol in at least any step of the step of preparing the aqueous phase, the step of preparing the oil phase, the step of mixing the aqueous phase and the oil phase together, and the step of mixing the water and the oil phase together.
(25) Use of a carotenoid and an emulsifier as an emulsion composition, wherein the emulsifier is any combination of the following (a), (b), (c), (d), (e), (f), (g), (h), (i), and (j):

(a) tetraglycerin monooleate and decaglycerin monolaurate;
(b) tetraglycerin monooleate and sucrose stearate;
(c) tetraglycerin monooleate and sucrose laurate;
(d) decaglycerin monolaurate and sucrose laurate;
(e) sucrose stearate and sucrose laurate;
(f) tetraglycerin monooleate, decaglycerin monolaurate, and sucrose stearate;
(g) tetraglycerin monooleate, decaglycerin monolaurate, and sucrose laurate;
(h) tetraglycerin monooleate, sucrose stearate, and sucrose laurate;
(i) decaglycerin monolaurate, sucrose stearate, and sucrose laurate; and
(j) tetraglycerin monooleate, decaglycerin monolaurate, sucrose stearate, and sucrose laurate.

(26) Use of a carotenoid and an emulsifier containing decaglycerin monolaurate and sucrose stearate as an emulsion composition, wherein the emulsion composition is free of lecithin.
(27) Use of an emulsion composition as a soft capsule formulation, wherein the emulsion composition contains a carotenoid and an emulsifier, the emulsifier being any combination of the following (a), (b), (c), (d), (e), (f), (g), (h), (i), and (j):

(a) tetraglycerin monooleate and decaglycerin monolaurate;

(b) tetraglycerin monooleate and sucrose stearate;

(c) tetraglycerin monooleate and sucrose laurate;

(d) decaglycerin monolaurate and sucrose laurate;

(e) sucrose stearate and sucrose laurate;

(f) tetraglycerin monooleate, decaglycerin monolaurate, and sucrose stearate;

(g) tetraglycerin monooleate, decaglycerin monolaurate, and sucrose laurate;

(h) tetraglycerin monooleate, sucrose stearate, and sucrose laurate;

(i) decaglycerin monolaurate, sucrose stearate, and sucrose laurate; and

(j) tetraglycerin monooleate, decaglycerin monolaurate, sucrose stearate, and sucrose laurate.

(28) Use of an emulsion composition as a soft capsule formulation, wherein the emulsion composition contains a carotenoid, and decaglycerin monolaurate and sucrose stearate, and the emulsion composition is free of lecithin.

Advantageous Effects of Invention

The emulsion composition according to the present invention is capable of enhancing systemic absorption for various carotenoids, well compatible with soft capsules, and thus capable of being formulated into a soft capsule formulation, and capable of homogeneously dispersing raw materials including carotenoids and emulsifiers in soft capsule formulations.

Brief Description of Drawings

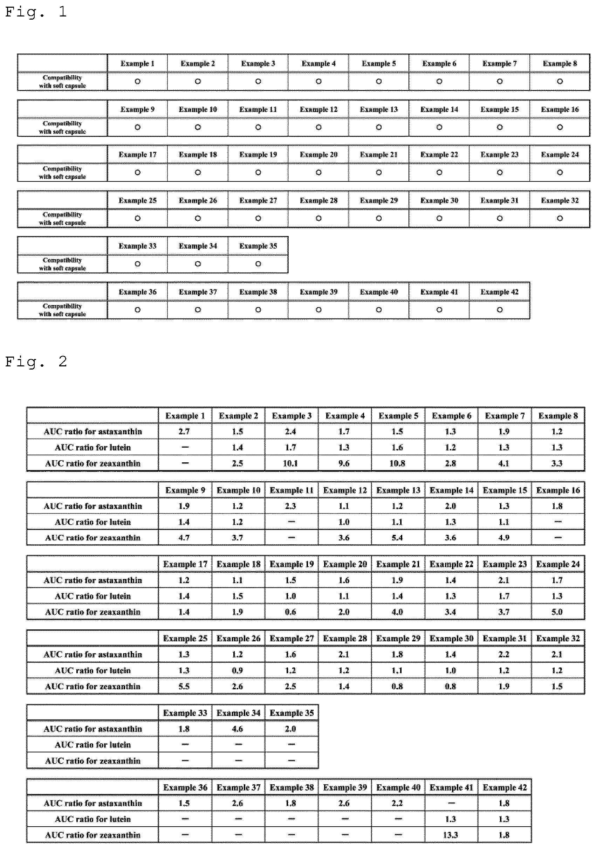

[Figure 1] Figure 1 shows tables showing results of evaluation of compatibility with a soft capsule (affinity with a soft capsule shell) for emulsion compositions according to Examples 1 to 42.
[Figure 2] Figure 2 shows tables showing ratios of area under the blood concentration-time curve ($AUC_{0-24hr}$) between a comparative composition and each of emulsion compositions according to Examples 1 to 42 for astaxanthin, lutein, and zeaxanthin.

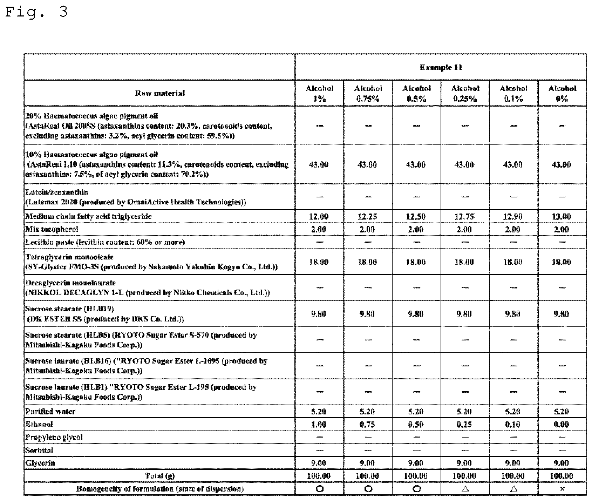

[Figure 3] Figure 3 shows a table showing formulation and results of evaluation of homogeneity for an emulsion composition according to Example 11 with alcohol (ethanol) concentrations of 0% (0% by weight), 0.1% (0.1% by weight), 0.25% (0.25% by weight), 0.5% (0.5% by weight), 0.75% (0.75% by weight), and 1% (1% by weight).

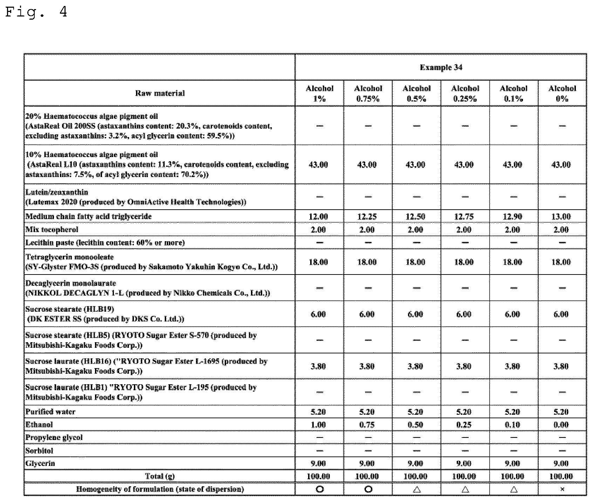

[Figure 4] Figure 4 shows a table showing formulation and results of evaluation of homogeneity for an emulsion composition according to Example 34 with alcohol (ethanol) concentrations of 0% (0% by weight), 0.1% (0.1% by weight), 0.25% (0.25% by weight), 0.5% (0.5% by weight), 0.75% (0.75% by weight), and 1% (1% by weight).

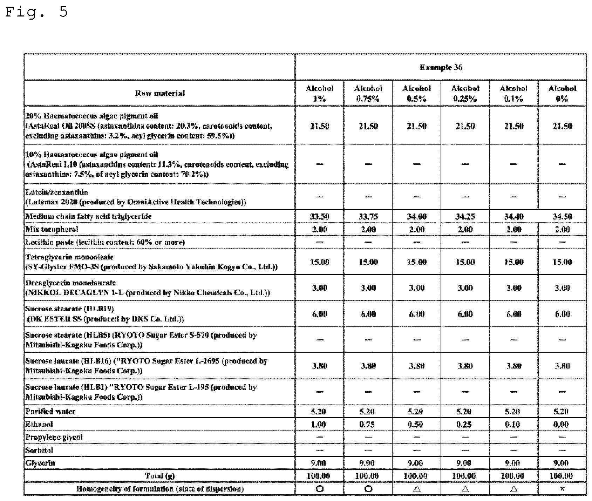

[Figure 5] Figure 5 shows a table showing formulation and results of evaluation of homogeneity for an emulsion composition according to Example 36 with alcohol (ethanol) concentrations of 0% (0% by weight), 0.1% (0.1% by weight), 0.25% (0.25% by weight), 0.5% (0.5% by weight), 0.75% (0.75% by weight), and 1% (1% by weight).

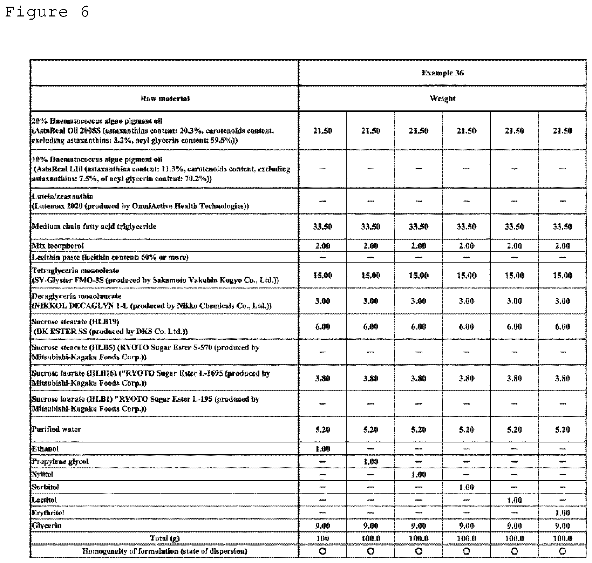

[Figure 6] Figure 6 shows a table showing formulation and results of evaluation of homogeneity for an emulsion composition according to Example 36 with addition of any of propylene glycol (dihydric alcohol), and (polyhydric) sugar alcohols including xylitol, sorbitol, lactitol, and erythritol, in place of ethanol (monohydric alcohol).

Description of Embodiments

Hereinafter, an emulsion composition according to the present invention and a production method therefor will be described in detail. The emulsion composition according to the present invention contains a carotenoid, and two or more specific emulsifiers selected from the group consisting of tetraglycerin monooleate, decaglycerin monolaurate (decaglyceryl monolaurate), sucrose stearate, and sucrose laurate.

The carotenoid refers to a group of compounds classified as one of terpenoids, and a collective term for aliphatic or alicyclic polyenes including many conjugated double bonds as yellow or red pigments (carotenoid pigments).

For the carotenoid applicable in the present invention, any carotenoid may be selected without limitation, unless the characteristics of the present invention are deteriorated, and examples such carotenoids can include hydrocarbons (carotenes) and oxidized alcohol derivatives thereof (xanthophylls), more specifically, actinioerythrol, bixin, canthaxanthin, capsanthin, capsorubin, β-8'-apo-carotenal (apocarotenal), β-12'-apocarotenal, α-carotene, β-carotene, carotene (a mixture of α- and β-carotenes), γ-carotene, β-cryptoxanthin, lutein, lycopene, violerythrin, zeaxanthin, fucoxanthin, phytoene, phytofluene, 3,4,3',4'-tetradehydrolycopene, torulene, diaponeurosporene, diapolycopene, diapolycopenedial, staphyloxanthin, crocetin, adonirubin, adonixanthin, echinenone, asteroidenone, and 3-hydroxyechinenone. The scope of the carotenoid in the present invention includes esters (fatty acid esters) and glycosides of carotenoids including a hydroxy group or carboxy group among the listed carotenoids. The carotenoid is not limited to naturally-occurring carotenoids, and synthesized products of carotenoids obtained in accordance with a conventional method are also included in the scope of the carotenoid in the present invention.

Among those carotenoids, one or more selected from the group consisting of lutein, zeaxanthin, astaxanthin, lycopene, β-carotene, γ-carotene, phytofluene, phytoene, canthaxanthin, β-cryptoxanthin, capsanthin, fucoxanthin, and fatty acid esters of them are preferred, and one or more selected from lutein, zeaxanthin, astaxanthin, and fatty acid esters of them are more preferred.

Lutein (β,ε-carotene-3,3'-diol) is known to be rich in green and yellow vegetables such as spinach, kale, and Japanese mustard spinach, and be rich, in the form of lutein fatty acid ester, in fruits such as orange, peach, papaya, prune, and mango, most flowers and vegetables, in particular, petals of marigold. Lutein is abundant in the macula in the retina and believed to function to exhibit protective action against light, and reported to serve for prevention of age-related macular degeneration, as well as protection of the macula. In addition, lutein is confirmed to have effects of antioxidative activity such as elimination activity for singlet oxygen, cancer prevention, and so on.

For the lutein applicable in the present invention, any lutein may be selected without limitation, unless the characteristics of the present invention are deteriorated, and examples of such lutein can include the above-described naturally-occurring lutein from green and yellow vegetables, flowers, vegetables, and so on, artificially produced lutein, and lutein obtained through gene recombination. Commercially available products of lutein may be used, and lutein in the form of a pharmacologically acceptable salt may be used.

Zeaxanthin (4-[18-(4-hydroxy-2,6,6-trimethyl-1-cyclohexenyl)-3,7,12,16-tetramethyl-octadeca-1,3,5,7,9,11,13,15,17-nonaenyl]-3,5,5-trimethyl-3-cyclohexen-1-ol), a fat-soluble substance similar to β-carotene, is a structural isomer of lutein. As with the case of lutein, zeaxanthin is abundant in the macular, and thus said to function to protect the macular like lutein.

For the zeaxanthin applicable in the present invention, any zeaxanthin may be selected without limitation, unless the characteristics of the present invention are deteriorated, and examples of such zeaxanthin can include naturally-occurring zeaxanthin from plants such as corn, yolk, animal fat, and so on, artificially produced zeaxanthin, and zeaxanthin obtained through gene recombination. Commercially available products of zeaxanthin may be used, and zeaxanthin in the form of a pharmacologically acceptable salt may be used.

Astaxanthin (3,3'-dihydroxy-β,β-carotene-4,4'-dione) is a red pigment used for a wide variety of foods and widely distributed in the nature, in particular, in oceans, and is derived from crustaceans such as shrimps and crabs, fishes such as salmons and sea breams, algae such as the green algae Haematococcus spp., yeasts such as the red yeast Phaffia. Astaxanthin is known to have potent antioxidative activity approximately 1000 times higher than that of vitamin E and 40 times higher than that of β-carotene, and have bioactivities including antioxidative effect, anti-inflammatory effect, antiaging effect for the skin, and whitening effect. In addition, astaxanthin is known as a pigment in the range of yellow to red. There are three isomers of astaxanthin, the 3S,3'S-form, the 3S,3'R-form (meso-form), and the 3R,3'R-form, which have different conformations of the hydroxy group at position 3(3') in the ring structure present at each end of the molecule. Further, there are geometric isomers of astaxanthin due to cis-/trans-configuration in conjugated double bonds in the center of the molecule. For example, there are the all-trans-form, the 9-cis-form, and the 13-cis-form. Moreover, the hydroxy group at position 3(3') is capable of forming an ester form with a fatty acid.

Further, astaxanthin is known as a highly safe compound for which no mutagenicity has been observed, and is widely used as a food additive (Takahashi, Jiro et al.: Toxicity Test of Haematococcus Algae Astaxanthin - Ames Test, Toxicity Test with Single Administration to Rats, Toxicity Test with Repetitive Oral Administration to Rats for 90 Days -, Journal of Clinical Therapeutics & Medicines, 20: 867-881, 2004).

The scope of the astaxanthin in the present invention includes free forms of astaxanthin and/or derivatives such as esters thereof. Esters of astaxanthin include monoester forms and/or diester forms. For example, astaxanthin obtained from Haematococcus pluvialis is known to be in the 3S,3'S-form, and molecules thereof are known to include many monoester forms with one fatty acid molecule bonding thereto (Renstrom, B. et al., Fatty acids of some esterified carotenols, Comp. Biochem. Physiol. B, Comp. Biochem., 1981, 69, p. 625-627). On the other hand, molecules of astaxanthin obtained from krill are known to include many diester forms with two fatty acid molecules bonding thereto (Yamaguchi, K. et al., The composition of carotenoid pigments in the Antarctic krill Euphausia superba, Bull. Jap. Sos. Sci. Fish., 1983, 49, p. 1411-1415) .

Astaxanthin obtained from Phaffia rhodozyma is known to be in the 3R,3'R-form (Andrewes, A. G. et al., (3R,3'R)-Astaxanthin from the yeast Phaffia rhodozyma, Phytochem., 1976, 15, p. 1009-1011), which has a structure reverse of that of the 3S,3'S-form, which is commonly found in the nature. The 3R,3'R-form is present as a non-ester form without forming an ester with a fatty acid, that is, in a free form (Andrewes, A. G. et al., Carotenids of Phaffia rhodozyma, a red pigmented fermenting yeast, Phytochem., 1976, 15, p. 1003-1007).

For the astaxanthin applicable in the present invention, any astaxanthin may be selected without limitation, unless the characteristics of the present invention are deteriorated, and examples of such astaxanthin can include natural astaxanthin and synthesized astaxanthin. Examples of natural astaxanthin can include astaxanthin-containing extracts themselves obtained from algae such as Haematococcus spp.; yeasts such as Phaffia sp.; crustaceans such as shrimps, krill, and crabs; cephalopods such as squids and octopuses; other fish and shellfish; plants such as Adonis spp.; bacteria such as Paracoccus sp.N81106, Brevundimonas sp.SD212, and Erythrobacter sp.PC6; actinomycetes such as Gordonia sp.KANMONKAZ-1129; labyrinthulomycetes such as Schizochytriuym sp.KH105; and gene recombinant astaxanthin-producing organisms; and astaxanthin appropriately purified from any of the astaxanthin-containing extracts. Preferred is astaxanthin derived from a microalgae extract extracted from microalgae such as Haematococcus spp., and more preferred is astaxanthin derived from a Haematococcus algae extract extracted from Haematococcus algae. Examples of synthesized astaxanthin can include AstaSana (produced by Koninklijke DSM N.V.) and Lucantin Pink (registered trademark; produced by BASF SE). Examples of synthesized astaxanthin obtained by chemically converting another naturally-occurring carotenoid can include AstaMarine (produced by PIVEG, Inc.).

Examples of Haematococcus algae that give natural astaxanthin can include Haematococcus pluvialis, Haematococcus lacustris, Haematococcus capensis, Haematococcus deroebakensis, and Haematococcus zimbabwiensis.

Preferred methods of culturing these Haematococcus green algae are closed culture methods, which are free from contamination with and reproduction of foreign microorganisms and allow less inclusion of other contaminants, and examples of such culture methods can include a method of culturing by using an incubator including a partially open, domed, conical, or cylindrical culture device and a gas jet unit freely movable in the device (International Publication No. WO 1999/050384), a method in which drought stress is applied to Haematococcus algae to induce encystment of the algae, and astaxanthin is collected from the culture of the encysted algae (Japanese Patent Laid-Open No. 8-103288), a method of culturing in a closed incubator with irradiation with light from a light source set in the inside of the incubator, and a method using a tabular culture tank or a tubular culture tank.

The astaxanthin applicable in the present invention may be an astaxanthin-containing extract obtained by crushing the cell walls of the above-described Haematococcus algae, for example, in accordance with a method disclosed in Japanese Patent Laid-Open No. 5-068585, as necessary, and extracting with addition of an organic solvent such as acetone, ether, chloroform, and alcohol (e.g., ethanol, methanol) or an extraction medium/solvent such as supercritical carbon dioxide; or a product obtained by appropriately purifying the astaxanthin-containing extract, as necessary. The astaxanthin content of the astaxanthin-containing extract is preferably 3 to 40% (w/w), more preferably 3 to 12% (w/w), and even more preferably 5 to 10% (w/w).

Examples of the astaxanthin applicable in the present invention can include commercially available products thereof. Examples of such commercially available products can include AstaReal, astavita, and astamate series such as AstaReal Oil 200SS, AstaReal L10, AstaReal Oil 50F, AstaReal Oil 50FC, AstaReal Oil 5F, AstaReal P2AF, AstaTROL-X, AstaReal Oil 50FC, AstaReal Powder 20F, Water-Soluble AstaReal Solution, AstaReal WS Solution, AstaReal 10WS Solution, AstaReal ACT, astavita e, astavita SPORTS, and astamate (all are registered trademarks; produced by AstaReal Co., Ltd., produced by Fuji Chemical Industries Co., Ltd.); ASTOTS series such as ASTOTS-S, ASTOTS-10O, ASTOTS-ECS, ASTPTS-2.0PW, and ASTOTS-3.0MB (all ASTOTS are registered trademarks; produced by FUJIFILM Corporation); BioAstin (registered trademark; produced by Cyanotech Corporation); Astazine (TM) (produced by BGG Japan); Astaxanthin Powder 1.5%, Astaxanthin Powder 2.5%, Astaxanthin Oil 5%, and Astaxanthin Oil 10% (Bio Actives Japan Corporation); ASTAXANTHIN (produced by Oryza Oil & Fat Chemical Co., Ltd.); SunActive AX (registered trademark; produced by Taiyo Kagaku Co., Ltd.); Haematococcus WS30 (produced by YAEGAKI Bio-industry, Inc.); and AstaMarine (produced by PIVEG, Inc.).

Each of AstaReal Oil 200SS, AstaReal Oil 50FC, AstaReal P2AF, AstaTROL-X, AstaReal Oil 50FC, and AstaReal Powder 20F produced by AstaReal Co., Ltd. and produced by Fuji Chemical Industries Co., Ltd. has acquired the "Halal certification", and each of AstaReal Oil 50FC, AstaReal P2AF, AstaTROL-X, AstaReal Oil 50FC, and AstaReal Powder 20F produced by AstaReal Co., Ltd. and produced by Fuji Chemical Industries Co., Ltd. has acquired the "Kosher certification". Moreover, AstaReal L10 has acquired Non-GMO (certification of non-genetically modified organisms).

Additionally, the emulsion composition according to the present invention contains an emulsifier (polyglycerin fatty acid ester and/or sucrose fatty acid ester) being any combination of the following (a), (b), (c), (d), (e), (f), (g), (h), (i), and (j):

   (a) tetraglycerin monooleate and decaglycerin monolaurate (decaglyceryl monolaurate);
   (b) tetraglycerin monooleate and sucrose stearate;
   (c) tetraglycerin monooleate and sucrose laurate;
   (d) decaglycerin monolaurate and sucrose laurate;
   (e) sucrose stearate and sucrose laurate;
   (f) tetraglycerin monooleate, decaglycerin monolaurate, and sucrose stearate;
   (g) tetraglycerin monooleate, decaglycerin monolaurate, and sucrose laurate;
   (h) tetraglycerin monooleate, sucrose stearate, and sucrose laurate;
   (i) decaglycerin monolaurate, sucrose stearate, and sucrose laurate; and
   (j) tetraglycerin monooleate, decaglycerin monolaurate, sucrose stearate, and sucrose laurate.

Examples of the tetraglycerin monooleate, decaglycerin monolaurate, sucrose stearate, and sucrose laurate applicable in the present invention can include commercially available products thereof. Examples of such commercially available products can include SY-Glyster FMO-3S (produced by Sakamoto Yakuhin Kogyo Co., Ltd.) for tetraglycerin monooleate; NIKKOL DECAGLYN 1-L (produced by Nikko Chemicals Co., Ltd.), RYOTO Polygly Ester L-10D (produced by Mitsubishi-Kagaku Foods Corp.), and SY-Glyster ML-750 (produced by Sakamoto Yakuhin Kogyo Co., Ltd.) for decaglycerin monolaurate; RYOTO Sugar Ester (S-070, S-170, S-270, S-370, S-370F, S-570, S-770, S-970, S-1170, S-1170F, S-1570, S-1670; produced by Mitsubishi-Kagaku Foods Corp.) and DK ESTER SS (produced by DKS Co. Ltd.) for sucrose stearate; and RYOTO Sugar Ester (L-195, L-595, L-1695, LWA-1570; produced by Mitsubishi-Kagaku Foods Corp.) and DK ESTER S-L18A (produced by DKS Co. Ltd.) for sucrose laurate.

The emulsion composition according to the present invention may contain an additional polyglycerin fatty acid ester differing from tetraglycerin monooleate and/or decaglycerin monolaurate. Any such polyglycerin fatty acid ester may be selected without limitation, unless the characteristics of the present invention are deteriorated, and examples thereof can include an ester of a polyglycerin with an average degree of polymerization of 2 or higher and citric acid, caprylic acid, capric acid, lauric acid, myristic acid, palmitic acid, stearic acid, oleic acid, linoleic acid, pentastearic acid, monoisostearic acid, diisostearic acid, pentaisostearic acid, or pentaoleic acid, more specifically, hexaglycerin monooleate, hexaglycerin monostearate, hexaglycerin monopalmitate, hexaglycerin monomyristate, hexaglycerin monolaurate, decaglycerin monooleate, decaglycerin monostearate, decaglycerin monopalmitate, decaglycerin monomyristate, decaglycerin monolaurate, glycerin stearate citrate, decaglycerin distearate, tetraglycerin monostearate, tetraglycerin tristearate, hexaglycerin monooleate, hexaglycerin monolaurate, hexaglycerin monomyristate, hexaglycerin monostearate, hexaglycerin tristearate, hexaglycerin monopalmitate, decaglycerin monomyristate, decaglycerin monostearate, decaglycerin monolaurate, decaglycerin monopalmitate, decaglycerin distearate, decaglycerin trioleate, decaglycerin tristearate, decaglycerin pentastearate, decaglycerin monoisostearate, decaglycerin diisostearate, decaglycerin pentaisostearate, and decaglycerin pentaoleate. One or more polyglycerin fatty acid esters selected from the group consisting of those polyglycerin fatty acid esters may be contained.

The emulsion composition according to the present invention may contain an additional sucrose fatty acid ester differing from sucrose stearate and/or sucrose laurate. Any such sucrose fatty acid ester may be selected without limitation, unless the characteristics of the present invention are deteriorated. Preferred are sucrose fatty acid esters having high hydrophilicity and superior water dispersibility, and examples thereof can include a sucrose fatty acid ester in which a fatty acid having 6 to 22 carbon atoms is bonding to one or more of the hydroxy groups in sucrose through an ester bond, more specifically, sucrose myristate, sucrose palmitate, sucrose oleate, and sucrose erucate. One or more sucrose fatty acid esters selected from the group consisting of those sucrose fatty acid esters may be contained.

Further, the emulsion composition according to the present invention may contain an emulsifier other than the above-described polyglycerin fatty acid ester and/or sucrose fatty acid ester. Any such emulsifier may be selected without limitation, unless the characteristics of the present invention are deteriorated, and examples thereof can include various emulsifiers conventionally used for foods and beverages, and so on, specifically, fatty acid monoglyceride, fatty acid diglyceride, fatty acid triglyceride, propylene glycol fatty acid ester, lecithin, chemically modified starches, sorbitan fatty acid ester, succinate fatty acid ester, Quillaja extract, gum arabic, gum tragacanth, guar gum, karaya gum, xanthan gum, pectin, alginic acid and salts thereof, carrageenan, gelatin, casein, saponin, and sterols. One or more emulsifiers selected from the group consisting of those emulsifiers may be contained.

If containing decaglycerin monolaurate and sucrose stearate, however, the emulsion composition according to the present invention is free of lecithin.

Additionally, the emulsion composition according to the present invention may contain an alcohol. Any alcohol may be selected without limitation, unless the characteristics of the present invention are deteriorated, and examples thereof can include lower alcohols, higher alcohols, and polyhydric alcohols, more specifically, monohydric alcohols such as ethanol, propanol, isopropanol, isobutyl alcohol, and t-butyl alcohol; dihydric alcohols such as ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol; trihydric alcohols such as glycerin and trimethylolpropane; tetrahydric alcohols such as pentaerythritol such as 1,2,6-hexanetriol; pentahydric alcohols such as xylitol; hexahydric alcohols such as sorbitol and mannitol; polyhydric alcohol polymers such as diethylene glycol, dipropylene glycol, triethylene glycol, polypropylene glycol, tetraethylene glycol, diglycerin, polyethylene glycol, triglycerin, tetraglycerin, and polyglycerin; higher alcohols such as batyl alcohol, cetanol, palmitoleyl alcohol, heptadecanol, 1-heptadecanol, stearyl alcohol, isostearyl alcohol, elaidyl alcohol, oleyl alcohol, linoleyl alcohol, elaidolinoleyl alcohol, linolenyl alcohol, elaidolinolenyl alcohol, ricinoleyl alcohol, nonadecyl alcohol, arachidyl alcohol, heneicosanol, behenyl alcohol, and erucyl alcohol; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono(2-methylhexyl) ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate; glycerin monoalkyl ethers; sugar alcohols such as sorbitol, maltitol, maltotriose, mannitol, sucrose, erythritol, glucose, fructose, starch sugar, maltose, xylitose, and reduced alcohol from starch sugar; Glysolid; and other alcohols including tetrahydrofurfuryl alcohol such as POE-tetrahydrofurfuryl alcohol, POP-butyl ether, POP/POE-butyl ether, tripolyoxypropylene glycerin ether, POP-glycerin ether, POP-glycerin ether phosphate, and POP/POE-pentane erythritol ether. One or more alcohols selected from the group consisting of those alcohols may be contained. Preferred are alcohols that enhance homogeneity of emulsifiers, and specifically, more preferred are monohydric alcohols such as methanol and ethanol, dihydric alcohols such as propylene glycol, and sugar alcohols such as xylitol, sorbitol, lactitol, and erythritol.

Additionally, the emulsion composition according to the present invention may contain a mineral. Any mineral may be selected without limitation, unless the characteristics of the present invention are deteriorated, and examples of thereof can include organic acid salts and inorganic acid salts, minerals blended as food additives typified by chemically synthesized products, and minerals blended from food materials such as extracts derived from various foods and yeasts, more specifically, calcium, phosphorus, copper, zinc, manganese, chromium, molybdenum, selenium, iron, sodium, potassium, magnesium, iodine, sodium phosphate, sodium hydrogen phosphate, sodium citrate, and potassium phosphate. One or more minerals selected from the group consisting of those minerals may be contained. Preferred are, however, zinc yeast and minerals derived from yeasts such as zinc yeast.

Additionally, the emulsion composition according to the present invention may contain a vitamin. Any vitamin may be selected without limitation, unless the characteristics of the present invention are deteriorated, and examples of thereof can include fat-soluble vitamins and water-soluble vitamins, specifically, vitamins A such as retinol, retinal, and retinoic acid, and derivatives thereof; vitamins D such as ergocalciferol and cholecalciferol; vitamins E such as tocopherol and tocotrienol; vitamins K such as phylloquinone and menaquinone; vitamins B1 such as thiamine hydrochloride; vitamins B2 such as riboflavin and flavin-adenine dinucleotide; niacins such as nicotinic acid and nicotinamide; pantothenic acid compounds such as pantothenic acid, calcium pantothenate, and pantothenyl alcohol (panthenol); vitamins B6 such as pyridoxine, pyridoxal, and pyridoxamine; biotin compounds such as biotin and biocytin; folic acid compounds such as folic acid; vitamins B12 such as cyanocobalamin; vitamins C (ascorbic acid and derivatives thereof) such as sodium ascorbyl phosphate, magnesium ascorbyl phosphate, ascorbyl glucoside, and ascorbyl tetra(2-hexyldecanoate); and other vitamin-like factors such as carnitine, ferulic acid, orotic acid, and $\gamma$-oryzanol. One or more vitamins selected from the group consisting of those vitamins may be contained. Preferred are, however, vitamins C and vitamins E.

Additionally, the emulsion composition according to the present invention may contain an oil or fat as a fat-soluble component. Any oil or fat may be selected without limitation, unless the characteristics of the present invention are deteriorated, and examples thereof can include oils, which are liquid at room temperature, and fats, which are solid at room temperature, and mixtures of them. Examples of liquid oils include olive oil, Camellia oil, macadamia nut oil, castor oil, avocado oil, evening primrose oil, turtle oil, corn oil, mink oil, rapeseed oil, egg-yolk oil, sesame oil, persic oil, wheat germ oil, Camellia sinensis leaf oil, linseed oil, safflower oil, cottonseed oil, perilla oil, soybean oil, peanut oil, Camellia sinensis seed oil, torreya seed oil, rice bran oil, Chinese paulownia oil, Japanese paulownia oil, jojoba oil, germ oil, glycerin trioctanoate, glycerin triisopalmitate, salad oil, safflower oil (Carthamus tinctorius oil), palm oil, coconut oil, peanut oil, almond oil, hazelnut oil, walnut oil, and grape seed oil. Examples of solid fats include beef tallow, hydrogenated beef tallow, neatsfoot oil, beef bone fat, mink oil, egg-yolk oil, lard, horse fat, mutton tallow, hydrogenated oil, cocoa butter, coconut oil, hydrogenated coconut oil, palm oil, hydrogenated palm oil, Japan wax, Japan wax kernel oil, and hydrogenated castor oil. One or more oils or fats selected from the group consisting of those oils and fats may be contained.

Further, medium chain fatty acid triglyceride is preferably used as the oil or fat. Medium chain fatty acid glyceride refers to a lipid formed by bonding a saturated fatty acid having 6 to 12 carbon atoms, specifically, any of caproic acid, caprylic acid, capric acid, and lauric acid to glycerol via an ester bond. The number of fatty acid molecules bonding to glycerol via an ester bond may be any of 1 to 3, and a mixture of medium chain fatty acid glycerides with different numbers of fatty acid molecules is acceptable. That is, monoglyceride, diglyceride, and triglyceride are all acceptable, and any mixture of them is also acceptable. Medium chain fatty acid glycerides with a high unsaturated fatty acid content (e.g., olive oil, safflower oil) are liquid at room temperature, and those with a high saturated fatty acid content (e.g., coconut oil, palm oil) are solid at room temperature. Medium chain fatty acid glycerides are contained in palm oil, coconut oil, and so on, and hence these are also preferably used. If a Haematococcus algae extract containing 18% by weight or more of astaxanthin is used, stable micelles can be formed by blending the above-described oil or fat.

Additionally, the emulsion composition according to the present invention may contain a radical scavenger. A radical scavenger is an excipient to play a role of preventing the generation of radicals and capturing generated radicals as quickly as possible to stop chain reaction (source: "The handbook of oil chemistry: lipids and surfactants, 4th Edition", Japan Oil Chemists' Society (Ed.), 2001). Known as a direct method for confirming functions as a radical scavenger is a method of mixing with a reagent and measuring capture of radicals by using a spectrometer or an ESR (electron spin resonance spectrometer).

Any compound applicable as a radical scavenger may be selected without limitation, unless the characteristics of the present invention are deteriorated, and compounds that function as a radical scavenger among antioxidants described in "Ko-Sanka-Zai no Riron to Jissai (Translated title: "Theory and Practical Use of Antioxidants", Kazimoto, Sanshu Shobo, 1984) and "Sanka-Boshi-Zai Handbook" (Translated title: "Handbook of Antioxidants", Saruwatari et al., TAISEISHA LTD., 1976) may be used. Specific examples thereof can include compounds having a phenolic hydroxy group; amine compounds including phenylenediamine such as diphenyl-p-phenylenediamine and 4-amino-p-diphenylamine; ascorbic acid; and oil-soluble derivatives of erythorbic acid, and specific examples of compounds having a phenolic hydroxy group can include gum guaiac; nordihydroguaiaretic acid (NDGA); gallates such as propyl gallate, butyl gallate, and octyl gallate; BHT (butylhydroxytoluene); BHA (butylhydroxyanisole); tocopherols such as mix tocopherol; and bisphenols. One or more compounds selected from the group consisting of those compounds applicable as a radical scavenger may be contained. However, tocopherols are preferred.

Additionally, the emulsion composition according to the present invention may contain a polyol. Any polyol may be selected without limitation, unless the characteristics of the present invention are deteriorated, and examples thereof include polyols that have viscosity-adjustment function and lower interfacial tension between water and oil or fat components, allowing the interface to easily spread to facilitate formation of a stable emulsion composition, more specifically, dihydric or higher-hydric alcohols such as glycerin, diglycerin, triglycerin, polyglycerin, 3-methyl-1,3-butanediol, 1,3-butylene glycol, isoprene glycol, polyethylene glycol, 1,2-pentanediol, 1,2-hexanediol, propylene glycol, dipropylene glycol, polypropylene glycol, ethylene glycol, diethylene glycol, pentaerythritol, neopentyl glycol, maltitol, reduced sugar syrup, fructose, glucose, sucrose, lactitol, Palatinit, erythritol, sorbitol, mannitol, xylitol, xylose, glucose, lactose, mannose, maltose, galactose, fructose, inositol, pentaerythritol, maltotriose, sorbitol, sorbitan, trehalose, starch sugar, and reduced alcohol from starch sugar. One of these polyols may be used singly, and one or more of them may be used in combination. However, it is preferred to contain one or more selected from glycerin, diglycerin, propylene glycol, ethylene glycol, 1,3-butylene glycol, polyethylene glycol, sorbitol, mannitol, dipropylene glycol, and sorbitan, and it is more preferred to contain at least glycerin.

Additionally, the emulsion composition of the present invention contains water in some cases. This water is not limited to particular water, and any water used for foods, drugs, and cosmetics, such as purified water, pure water, ion-exchanged water, alkaline ionized water, deep seawater, vibrated water, and natural water, may be used.

Further, the emulsion composition according to the present invention may contain, unless the characteristics of the present invention are deteriorated, any substance, for example, any of glycerophospholipids such as phosphatidic acid, bisphosphatidic acid, lecithin (phosphatidylcholine), phosphatidylethanolamine, phosphatidylmethylethanolamine, phosphatidylserine, phosphatidylinositol, phosphatidylglycerin, and diphosphatidylglycerin (cardiolipin), and hydrogenated or hydroxylated products of them; lyso compounds such as lysolecithin, lysophosphatidic acid, lysophosphatidylglycerin, lysophosphatidylinositol, lysophosphatidylethanolamine, lysophosphatidylmethylethanolamine, lysophosphatidylcholine (lysolecithin), and lysophosphatidylserine; lecithins derived from plants such as soybean, corn, peanut, rapeseed, and wheat, those derived from animals such as egg yolk and bovines, and those derived from microorganisms such as Escherichia coli; sphingophospholipids such as sphingomyelin (phospholipids, up to here); ascorbic acid and ascorbic acid derivatives and salts thereof such as L-ascorbic acid, sodium L-ascorbate, potassium L-ascorbate, calcium L-ascorbate, L-ascorbyl phosphate, magnesium L-ascorbyl phosphate, L-ascorbyl sulfate, disodium L-ascorbyl sulfate, and L-ascorbyl 2-glucoside; erythorbic acid and erythorbic acid derivatives and salts thereof such as erythorbic acid, sodium erythorbate, potassium erythorbate, calcium erythorbate, erythorbyl phosphate, and erythorbyl sulfate; flavonoids (e.g., catechin, anthocyanin, flavone, isoflavone, flavane, flavanone, rutin, glycosides thereof), phenols (e.g., chlorogenic acid, ellagic acid, gallic acid, propyl gallate), and polyphenols such as lignans, curcumins, and coumarins (antioxidants, up to here); and other flavors, sweeteners, acidulants, and coloring agents. One of these may be used singly, and one or more of them may be used in combination thereof.

Additionally, the emulsion composition of the present invention may have a self-emulsifying property. Self-emulsification in the present invention, which is also called spontaneous emulsification, refers to spontaneous formation of an emulsion through contact with an aqueous medium or digestive juice, without need of external force. If the emulsion composition of the present invention has a self-emulsifying property, the emulsion composition is capable of forming an emulsion stable in solutions containing more than 40%, 50%, 60%, 70%, 75%, 80%, 85%, or 90%(w/w) of water. In one preferred mode of the present invention, the water content is about 0.01 to 7 parts by weight to 100 parts by weight of the total of the emulsion composition.

A method for producing the emulsion composition according to the present invention includes the following steps (i) to (iii): (i) mixing and dissolving at least sucrose fatty acid ester, and, as necessary, an additional water-soluble substance such as a water-soluble emulsifier, a polyol, a water-soluble mineral, and a water-soluble vitamin in water to obtain an aqueous phase (aqueous phase preparation step); (ii) mixing and dissolving at least a carotenoid and a sucrose fatty acid ester and/or polyglycerin fatty acid ester, and, as necessary, an additional fat-soluble substance such as a fat-soluble emulsifier, an oil or fat, a compound applicable as a radical scavenger, a fat-soluble mineral, and a fat-soluble vitamin to obtain an oil phase (oil phase preparation step); and (iii) subsequently mixing the aqueous phase and the oil phase together to obtain a mixed solution and subjecting the resulting mixed solution to emulsification and dispersion to obtain an emulsion composition (emulsion composition preparation step).

When no sucrose fatty acid ester is used (mixed/dissolved) in aqueous phase preparation step (i), aqueous phase preparation step (i) is a step of mixing and dissolving water, and, as necessary, an additional water-soluble substance such as a water-soluble emulsifier, a polyol, a water-soluble mineral, and a water-soluble vitamin to obtain an aqueous phase, and emulsion composition preparation step (iii) is a step of mixing water or the aqueous phase and the oil phase together to obtain an emulsion composition.

When no polyglycerin fatty acid ester is used (mixed/dissolved) in oil phase preparation step (ii), oil phase preparation step (ii) is a step of mixing and dissolving at least a carotenoid and an oil or fat, and, as necessary, a sucrose fatty acid ester and an additional fat-soluble substance such as a fat-soluble emulsifier, a compound applicable as a radical scavenger, a fat-soluble mineral, and a fat-soluble vitamin to obtain an oil phase.

When at least sucrose stearate is mixed and dissolved in water in aqueous phase preparation step (i) and at least a carotenoid and decaglycerin monolaurate are mixed and dissolved in oil phase preparation step (ii), or when water, and, as necessary, an additional water-soluble substance such as a water-soluble emulsifier, a polyol, a water-soluble mineral, and a water-soluble vitamin are mixed, and at least a carotenoid, sucrose stearate, and decaglycerin monolaurate are mixed in oil phase preparation step (ii), lecithin is not mixed and dissolved in oil phase preparation step (ii).

Herein, an HLB (Hydrophile-Lipophile Balance) value may be used as an index to determine whether to use (mix and dissolve) a sucrose fatty acid ester in the aqueous phase preparation step or in the oil phase preparation step.

In this case, a commercially available product is used when a component to be used is available as a commercially available product, and if the HLB value of the commercially available product is clearly shown in a document such as a catalog of commercially available products, the HLB value shown in the document such as a catalog is employed.

If a component to be used is not available as a commercially available product, or if a component to be used is available as a commercially available product but the HLB value is not clearly shown in a document such as a catalog, on the other hand, a value determined by using Griffin's calculation formula is employed as an HLB value herein. Griffin's calculation formula uses the value S (saponification value of ester) and the value N (neutralization number of fatty acid constituting ester) for calculation of HLB values. The formula is as follows.

$$\text{HLB Value} = 20\ (1 - S/N)$$

An HLB value indicates higher hydrophilicity as the HLB value approaches 20, and higher lipophilicity as the HLB value approaches 0. Commonly, emulsifiers having an HLB value of lower than 7 are defined as being lipophilic, and those having an HLB value of 7 or higher are defined as being hydrophilic.

Emulsification and dispersion in emulsion composition preparation step (iii) may be performed by using a common emulsifier such as a stirrer, Impeller stirring, a homomixer, and a continuous flow shearing machine.

Additionally, a soft capsule formulation may be selected as a dosage form of the emulsion composition according to the present invention, and the soft capsule formulation is obtained by enclosing a content containing the emulsion composition according to the present invention in a soft capsule shell.

In the present invention, any soft capsule formulation may be selected without limitation, unless the characteristics of the present invention are deteriorated, and examples of such soft capsule formulations can include a capsule molded into a single container carrying a liquid payload or semisolid containing a drug, one or two or more excipients, and an optional diluent (e.g., including an emulsion formulation formed through self-emulsification and non-emulsion formulation).

A method for producing the soft capsule formulation according to the present invention includes obtaining the emulsion composition according to (1) or (2) in the above-described manner, and further includes the step of (iv) subsequently enclosing a content containing the thus-obtained emulsion composition in a soft capsule shell (soft capsule enclosure step).

An exemplary mode of soft capsule enclosure step (iv) in the present invention includes the following steps (1) to (3): (1) preparing a capsule content and a base material to form a soft capsule shell (material preparation step); (2) enclosing the content in the resulting base material to form a soft capsule shell, molding the base material, and drying the resultant to obtain an intermediate formulation (molding step); and (3) drying the resulting intermediate formulation (drying step).

"Material preparation step" (1) is a step of preparing a content containing the emulsion composition according to the present invention and a base material to form a soft capsule shell, and the step is as described above.

"Molding step" (2) is a step of enclosing the content containing the emulsion composition according to the present invention in a shell formed with the base material to form a soft capsule shell, molding the base material, and drying the resultant to obtain an intermediate formulation. A known method for producing a soft capsule formulation may be appropriately selected for enclosure of the content in the base material to form a soft capsule shell. Examples of such methods can include a plate method and a rotary die method.

"Drying step" (3) is a step of drying the intermediate formulation obtained in the molding step, and a soft capsule formulation is obtained through this step. A known drying means may be appropriately selected as a drying means used in the drying step. Examples of such means can include means with a known dryer such as a tumbler dryer (rotary drum dryer). Conditions including temperature and time in the drying step are not limited to particular conditions, and those according to the content of the capsule and the type of the base material to form a soft capsule shell may be appropriately selected.

In the present invention, any base material to form a soft capsule shell may be appropriately selected, unless the characteristics of the present invention are deteriorated, and examples thereof can include base materials containing as a main component any of the followings: gelatins such as gelatin, acidic gelatin, alkaline gelatin, peptide gelatin, low-molecular-weight gelatin, and gelatin derivatives; agar; and gellan gum. In the case that gelatin is used as a base material of a soft capsule shell, the base material is prepared from a gelatin shell solution containing gelatin, a plasticizer, and water. The soft capsule shell may contain a plasticizer, unless the characteristics of the present invention are deteriorated, and examples of such plasticizers can include glycerin; saccharides such as corn syrup, sucrose, fructose, sorbitol, and mannitol; glycols such as propylene glycol and polyethylene glycol; and water-insoluble celluloses such as microcrystalline cellulose, starches, low-substituted hydroxypropylcellulose, and ethylcellulose. One of these plasticizers may be used singly, and one or more of them may be used in combination thereof.

In the present invention, the content in the soft capsule formulation may contain 0.0001% by mass to 98% by mass of the emulsion composition according to the present invention to the total amount of the soft capsule formulation. The shape of the soft capsule formulation is not limited to a particular shape, and examples thereof can include an oval, an oblong, and a round. To form any of these shapes, a technique, an apparatus, and so on well-known in the art may be appropriately applied.

In the present invention, an excipient component commonly used in the field of foods and beverages and drugs may be appropriately blended in the soft capsule formulation (the content and/or the shell), unless the characteristics of the present invention are deteriorated. For example, an excipient component may be appropriately selected for blending from coloring agents such as dyes and pigments, flavors, sweeteners, taste-masking agents, odor-masking agents, preservatives, fruit juices, vitamins, animal and plant extracts, amino acids, minerals, thickeners, pH-adjusting agents, antiseptics, disintegrants, surfactants, and organic acids.

Examples

Hereinafter, the emulsion composition according to the present invention and the production method therefor will be described on the basis of Examples. It should be noted that the technical scope of the present invention is not limited to embodiments shown in Examples.

1. Preparation of Emulsion Composition

First, emulsion compositions according to Examples 1 to 42 were prepared as described below. In preparation of emulsion compositions according to Examples 1 to 42, "AstaReal Oil 200SS (produced by AstaReal Co., Ltd.)" was used as "20% Haematococcus algae pigment oil", "AstaReal L10 (produced by AstaReal Co., Ltd.)" was used as "10% Haematococcus algae pigment oil", and "Lutemax 2020 (produced by OmniActive Health Technologies)" was used as "lutein/zeaxanthin". "SY-Glyster FMO-3S (produced by Sakamoto Yakuhin Kogyo Co., Ltd.)" was used as "tetraglycerin monooleate", "NIKKOL DECAGLYN 1-L (produced by Nikko Chemicals Co., Ltd.)" was used as "decaglycerin monolaurate (decaglyceryl monolaurate)", "DK ESTER SS (produced by DKS Co. Ltd.)" and "RYOTO Sugar Ester S-570 (produced by Mitsubishi-Kagaku Foods Corp.)" were used as "sucrose stearate", and "RYOTO Sugar Ester L-1695 (produced by Mitsubishi-Kagaku Foods Corp.)" and "RYOTO Sugar Ester L-195 (produced by Mitsubishi-Kagaku Foods Corp.)" were used as "sucrose laurate". MCT oil was used as medium chain fatty acid triglyceride.

[1-1] Preparation of Emulsion Composition According to Example 1

An oil phase was prepared by adding 43 g of 10% Haematococcus algae pigment oil, 12 g of triglyceride, 2 g of mix tocopherol, and 18 g of tetraglycerin monooleate, and mixing and dissolving the resultant. Subsequently, 9.8 g of sucrose laurate having an HLB value of 16, 1 g of ethanol, and 9 g of glycerin were added to 5.2 g of purified water and dissolved to prepare an aqueous phase, and the oil phase and the aqueous phase were then mixed together to obtain an emulsion composition, which was used as an emulsion composition according to Example 1.

[1-2] Preparation of Emulsion Composition According to Example 2

An oil phase was prepared by adding 32 g of 10% Haematococcus algae pigment oil, 20 g of lutein/zeaxanthin oil, 0.8 g of triglyceride, 2 g of mix tocopherol, and 18 g of tetraglycerin monooleate, and mixing and dissolving the resultant. Subsequently, 13 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 2.

[1-3] Preparation of Emulsion Composition According to Example 3

An oil phase was prepared by adding 32 g of 10% Haematococcus algae pigment oil, 20 g of lutein/zeaxanthin oil, 3.8 g of triglyceride, 2 g of mix tocopherol, and 18 g of tetraglycerin monooleate, and mixing and dissolving the resultant. Subsequently, 10 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 3.

[1-4] Preparation of Emulsion Composition According to Example 4

An oil phase was prepared by adding 32 g of 10% Haematococcus algae pigment oil, 20 g of lutein/zeaxanthin oil, 7.8 g of triglyceride, 2 g of mix tocopherol, and 18 g of tetraglycerin monooleate, and mixing and dissolving the resultant. Subsequently, 6 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 4.

[1-5] Preparation of Emulsion Composition According to Example 5

An oil phase was prepared by adding 32 g of 10% Haematococcus algae pigment oil, 20 g of lutein/zeaxanthin oil, 11.8 g of triglyceride, 2 g of mix tocopherol, and 18 g of tetraglycerin monooleate, and mixing and dissolving the resultant. Subsequently, 2 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 5.

[1-6] Preparation of Emulsion Composition According to Example 6

An oil phase was prepared by adding 32 g of 10% Haematococcus algae pigment oil, 20 g of lutein/zeaxanthin oil, 10.8 g of triglyceride, 2 g of mix tocopherol, and 13.5 g of tetraglycerin monooleate, and mixing and dissolving the resultant. Subsequently, 7.5 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 6.

[1-7] Preparation of Emulsion Composition According to Example 7

An oil phase was prepared by adding 32 g of 10% Haematococcus algae pigment oil, 20 g of lutein/zeaxanthin oil, 9.8 g of triglyceride, 2 g of mix tocopherol, and 12 g of tetraglycerin monooleate, and mixing and dissolving the resultant. Subsequently, 10 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 7.

[1-8] Preparation of Emulsion Composition According to Example 8

An oil phase was prepared by adding 32 g of 10% Haematococcus algae pigment oil, 20 g of lutein/zeaxanthin oil, 17.8 g of triglyceride, 2 g of mix tocopherol, and 9 g of tetraglycerin monooleate, and mixing and dissolving the resultant. Subsequently, 5 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 8.

[1-9] Preparation of Emulsion Composition According to Example 9

An oil phase was prepared by adding 32 g of 10% Haematococcus algae pigment oil, 20 g of lutein/zeaxanthin oil, 15.8 g of triglyceride, 2 g of mix tocopherol, and 6 g of tetraglycerin monooleate, and mixing and dissolving the resultant. Subsequently, 10 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 9.

[1-10] Preparation of Emulsion Composition According to Example 10

An oil phase was prepared by adding 32 g of 10% Haematococcus algae pigment oil, 20 g of lutein/zeaxanthin oil, 3.8 g of triglyceride, 2 g of mix tocopherol, 18 g of tetraglycerin monooleate, and 10 g of sucrose laurate having an HLB value of 1, and mixing and dissolving the resultant. Subsequently, 6 g of propylene glycol and 3 g of glycerin were added to 5.2 g of purified water and dissolved to prepare an aqueous phase, and the oil phase and the aqueous phase were then mixed together to obtain an emulsion composition, which was used as an emulsion composition according to Example 10. Table 1 shows the formulations of the above emulsion compositions according to Examples 1 to 10.

[Table 1]

| Raw material | Example 1 Weight | Example 2 Weight | Example 3 Weight | Example 4 Weight | Example 5 Weight | Example 6 Weight | Example 7 Weight | Example 8 Weight | Example 9 Weight | Example 10 Weight |
|---|---|---|---|---|---|---|---|---|---|---|
| 20% Haematococcus algae pigment oil (AstaReal Oil 200SS (astaxanthins content: 20.3%, carotenoids content, excluding astaxanthins: 3.2%, acyl glycerin content: 59.5%)) | — | — | — | — | — | — | — | — | — | — |
| 10% Haematococcus algae pigment oil (AstaReal L10 (astaxanthins content: 11.3%, carotenoids content, excluding astaxanthins: 7.5%, of acyl glycerin content: 70.2%)) | 43 | 32 | 32 | 32 | 32 | 32 | 32 | 32 | 32 | 32 |
| Lutein/zeaxanthin (Lutemax 2020 (produced by OmniActive Health Technologies)) | — | 20 | 20 | 20 | 20 | 20 | 20 | 20 | 20 | 20 |
| Medium chain fatty acid triglyceride | 12 | 0.8 | 3.8 | 7.8 | 11.8 | 10.8 | 9.8 | 17.8 | 15.8 | 3.8 |
| Mix tocopherol | 2 | 2 | 2 | 2 | 2 | 2 | 2 | 2 | 2 | 2 |
| Lecithin paste (lecithin content: 60% or more) | — | — | — | — | — | — | — | — | — | — |
| Tetraglycerin monooleate (SY-Glyster FMO-3S (produced by Sakamoto Yakuhin Kogyo Co., Ltd.)) | 18 | 18 | 18 | 18 | 18 | 13.5 | 12 | 9 | 6 | 18 |
| Decaglycerin monolaurate (NIKKOL DECAGLYN 1-L (produced by Nikko Chemicals Co., Ltd.)) | — | — | — | — | — | — | — | — | — | — |
| Sucrose stearate (HLB19) (DK ESTER SS (produced by DKS Co. Ltd.)) | — | — | — | — | — | — | — | — | — | — |
| Sucrose stearate (HLB5) (RYOTO Sugar Ester S-570 (produced by Mitsubishi-Kagaku Foods Corp.)) | — | — | — | — | — | — | — | — | — | — |
| Sucrose laurate (HLB16) ("RYOTO Sugar Ester L-1695 (produced by Mitsubishi-Kagaku Foods Corp.)) | 9.8 | 13 | 10 | 6 | 2 | 7.5 | 10 | 5 | 10 | — |
| Sucrose laurate (HLB1) "RYOTO Sugar Ester L-195 (produced by Mitsubishi-Kagaku Foods Corp.)) | — | — | — | — | — | — | — | — | — | 10 |
| Purified water | 5.2 | 5.2 | 5.2 | 5.2 | 5.2 | 5.2 | 5.2 | 5.2 | 5.2 | 5.2 |
| Ethanol | 1 | — | — | — | — | — | — | — | — | — |
| Propylene glycol | — | 6 | 6 | 6 | 6 | 6 | 6 | 6 | 6 | 6 |
| Glycerin | 9 | 3 | 3 | 3 | 3 | 3 | 3 | 3 | 3 | 3 |
| Total (g) | 100 | 100 | 100 | 100 | 100 | 100 | 100 | 100 | 100 | 100 |

[1-11] Preparation of Emulsion Composition According to Example 11

An oil phase was prepared by adding 43 g of 10% Haematococcus algae pigment oil, 12 g of triglyceride, 2 g of mix tocopherol, and 18 g of tetraglycerin monooleate, and mixing and dissolving the resultant. Subsequently, 9.8 g of sucrose stearate having an HLB value of 19, 1 g of ethanol, and 9 g of glycerin were added to 5.2 g of purified water and dissolved to prepare an aqueous phase, and the oil phase and the aqueous phase were then mixed together to obtain an emulsion composition, which was used as an emulsion composition according to Example 11.

[1-12] Preparation of Emulsion Composition According to Example 12

An oil phase was prepared by adding 32 g of 10% Haematococcus algae pigment oil, 20 g of lutein/zeaxanthin oil, 7.8 g of triglyceride, 2 g of mix tocopherol, and 18 g of tetraglycerin monooleate, and mixing and dissolving the resultant. Subsequently, 6 g of sucrose stearate having an HLB value of 19, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 12.

[1-13] Preparation of Emulsion Composition According to Example 13

An oil phase was prepared by adding 32 g of 10% Haematococcus algae pigment oil, 20 g of lutein/zeaxanthin oil, 11.8 g of triglyceride, 2 g of mix tocopherol, and 18 g of tetraglycerin monooleate, and mixing and dissolving the resultant. Subsequently, 2 g of sucrose stearate having an HLB value of 19, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 13.

[1-14] Preparation of Emulsion Composition According to Example 14

An oil phase was prepared by adding 32 g of 10% Haematococcus algae pigment oil, 20 g of lutein/zeaxanthin oil, 9.8 g of triglyceride, 2 g of mix tocopherol, and 12 g of tetraglycerin monooleate, and mixing and dissolving the resultant. Subsequently, 10 g of sucrose stearate having an HLB value of 19, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 14.

[1-15] Preparation of Emulsion Composition According to Example 15

An oil phase was prepared by adding 32 g of 10% Haematococcus algae pigment oil, 20 g of lutein/zeaxanthin oil, 15.8 g of triglyceride, 2 g of mix tocopherol, and 6 g of tetraglycerin monooleate, and mixing and dissolving the resultant. Subsequently, 10 g of sucrose stearate having an HLB value of 19, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 15. shows the formulations of the above emulsion compositions according to Examples 11 to 15.

[Table 2]

| Raw material | Example 11 Weight | Example 12 Weight | Example 13 Weight | Example 14 Weight | Example 15 Weight |
|---|---|---|---|---|---|
| 20% Haematococcus algae pigment oil (AstaReal Oil 200SS (astaxanthins content: 20.3%, carotenoids content, excluding astaxanthins: 3.2%, acyl glycerin content: 59.5%)) | — | — | — | — | — |
| 10% Haematococcus algae pigment oil (AstaReal L10 (astaxanthins content: 11.3%, carotenoids content, excluding astaxanthins: 7.5%, of acyl glycerin content: 70.2%)) | 43 | 32 | 32 | 32 | 32 |
| Lutein/zeaxanthin (Lutemax 2020 (produced by OmniActive Health Technologies)) | — | 20 | 20 | 20 | 20 |
| Medium chain fatty acid triglyceride | 12 | 7.8 | 11.8 | 9.8 | 15.8 |
| Mix tocopherol | 2 | 2 | 2 | 2 | 2 |
| Lecithin paste (lecithin content: 60% or more) | — | — | — | — | — |
| Tetraglycerin monooleate (SY-Glyster FMO-3S (produced by Sakamoto Yakuhin Kogyo Co., Ltd.)) | 18 | 18 | 18 | 12 | 6 |
| Decaglycerin monolaurate (NIKKOL DECAGLYN 1-L (produced by Nikko Chemicals Co., Ltd.)) | — | — | — | — | — |
| Sucrose stearate (HLB19) (DK ESTER SS (produced by DKS Co. Ltd.)) | 9.8 | 6 | 2 | 10 | 10 |
| Sucrose stearate (HLB5) (RYOTO Sugar Ester S-570 (produced by Mitsubishi-Kagaku Foods Corp.)) | — | — | — | — | — |
| Sucrose laurate (HLB16) ("RYOTO Sugar Ester L-1695 (produced by Mitsubishi-Kagaku Foods Corp.)) | — | — | — | — | — |
| Sucrose laurate (HLB1) "RYOTO Sugar Ester L-195 (produced by Mitsubishi-Kagaku Foods Corp.)) | — | — | — | — | — |
| Purified water | 5.2 | 5.2 | 5.2 | 5.2 | 5.2 |
| Ethanol | 1 | — | — | — | — |
| Propylene glycol | — | 6 | 6 | 6 | 6 |
| Glycerin | 9 | 3 | 3 | 3 | 3 |
| Total (g) | 100 | 100 | 100 | 100 | 100 |

[1-16] Preparation of Emulsion Composition According to Example 16

An oil phase was prepared by adding 43 g of 10% Haematococcus algae pigment oil, 12 g of triglyceride, 2 g of mix tocopherol, 18 g of tetraglycerin monooleate, and 9.8 g of decaglycerin monolaurate, and mixing and dissolving the resultant. Subsequently, 1 g of ethanol and 9 g of glycerin were added to 5.2 g of purified water and dissolved to prepare an aqueous phase, and the oil phase and the aqueous phase were then mixed together to obtain an emulsion composition, which was used as an emulsion composition according to Example 16.

[1-17] Preparation of Emulsion Composition According to Example 17

An oil phase was prepared by adding 32 g of 10% Haematococcus algae pigment oil, 21 g of lutein/zeaxanthin oil, 1 g of triglyceride, 3 g of mix tocopherol, 18 g of tetraglycerin monooleate, and 9.8 g of decaglycerin monolaurate, and mixing and dissolving the resultant. Subsequently, 1 g of ethanol and 9 g of glycerin were added to 5.2 g of purified water and dissolved to prepare an aqueous phase, and the oil phase and the aqueous phase were then mixed together to obtain an emulsion composition, which was used as an emulsion composition according to Example 17.

[1-18] Preparation of Emulsion Composition According to Example 18

An oil phase was prepared by adding 32 g of 10% Haematococcus algae pigment oil, 21 g of lutein/zeaxanthin oil, 1 g of triglyceride, 3 g of mix tocopherol, 18 g of tetraglycerin monooleate, and 9.8 g of decaglycerin monolaurate, and mixing and dissolving the resultant. Subsequently, 1 g of propylene glycol and 9 g of glycerin were added to 5.2 g of purified water and dissolved to prepare an aqueous phase, and the oil phase and the aqueous phase were then mixed together to obtain an emulsion composition, which was used as an emulsion composition according to Example 18.

[1-19] Preparation of Emulsion Composition According to Example 19

An oil phase was prepared by adding 24 g of 10% Haematococcus algae pigment oil, 15.5 g of lutein/zeaxanthin oil, 2.3 g of triglyceride, 2 g of mix tocopherol, 27 g of tetraglycerin monooleate, and 15 g of decaglycerin monolaurate, and mixing and dissolving the resultant. Subsequently, 6 g of propylene glycol and 3 g of glycerin were added to 5.2 g of purified water and dissolved to prepare an aqueous phase, and the oil phase and the aqueous phase were then mixed together to obtain an emulsion composition, which was used as an emulsion composition according to Example 19. shows the formulations of the above emulsion compositions according to Examples 16 to 19.

[Table 3]

| Raw material | Example 16 | Example 17 | Example 18 | Example 19 |
|---|---|---|---|---|
| | Weight | Weight | Weight | Weight |
| 20% Haematococcus algae pigment oil (AstaReal Oil 200SS (astaxanthins content: 20.3%, carotenoids content, excluding astaxanthins: 3.2%, acyl glycerin content: 59.5%)) | — | — | — | — |
| 10% Haematococcus algae pigment oil (AstaReal L10 (astaxanthins content: 11.3%, carotenoids content, excluding astaxanthins: 7.5%, of acyl glycerin content: 70.2%)) | 43 | 32 | 32 | 24 |
| Lutein/zeaxanthin (Lutemax 2020 (produced by OmniActive Health Technologies)) | — | 21 | 21 | 15.5 |
| Medium chain fatty acid triglyceride | 12 | 1 | 1 | 2.3 |
| Mix tocopherol | 2 | 3 | 3 | 2 |
| Lecithin paste (lecithin content: 60% or more) | — | — | — | — |
| Tetraglycerin monooleate (SY-Glyster FMO-3S (produced by Sakamoto Yakuhin Kogyo Co., Ltd.)) | 18 | 18 | 18 | 27 |
| Decaglycerin monolaurate (NIKKOL DECAGLYN 1-L (produced by Nikko Chemicals Co., Ltd.)) | 9.8 | 9.8 | 9.8 | 15 |
| Sucrose stearate (HLB19) (DK ESTER SS (produced by DKS Co. Ltd.)) | — | — | — | — |
| Sucrose stearate (HLB5) (RYOTO Sugar Ester S-570 (produced by Mitsubishi-Kagaku Foods Corp.)) | — | — | — | — |
| Sucrose laurate (HLB16) ("RYOTO Sugar Ester L-1695 (produced by Mitsubishi-Kagaku Foods Corp.)) | — | — | — | — |
| Sucrose laurate (HLB1) "RYOTO Sugar Ester L-195 (produced by Mitsubishi-Kagaku Foods Corp.)) | — | — | — | — |
| Purified water | 5.2 | 5.2 | 5.2 | 5.2 |
| Ethanol | 1 | 1 | — | — |
| Propylene glycol | — | — | 1 | 6 |
| Glycerin | 9 | 9 | 9 | 3 |
| Total (g) | 100 | 100 | 100 | 100 |

[1-20] Preparation of Emulsion Composition According to Example 20

An oil phase was prepared by adding 32 g of 10% Haematococcus algae pigment oil, 20 g of lutein/zeaxanthin oil, 6.8 g of triglyceride, 2 g of mix tocopherol, and 15 g of decaglycerin monolaurate, and mixing and dissolving the resultant. Subsequently, 10 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 20.

[1-21] Preparation of Emulsion Composition According to Example 21

An oil phase was prepared by adding 32 g of 10% Haematococcus algae pigment oil, 20 g of lutein/zeaxanthin oil, 10.8 g of triglyceride, 2 g of mix tocopherol, and 15 g of decaglycerin monolaurate, and mixing and dissolving the resultant. Subsequently, 6 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 21.

[1-22] Preparation of Emulsion Composition According to Example 22

An oil phase was prepared by adding 32 g of 10% Haematococcus algae pigment oil, 20 g of lutein/zeaxanthin oil, 14.8 g of triglyceride, 2 g of mix tocopherol, and 15 g of decaglycerin monolaurate, and mixing and dissolving the resultant. Subsequently, 2 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 22.

[1-23] Preparation of Emulsion Composition According to Example 23

An oil phase was prepared by adding 32 g of 10% Haematococcus algae pigment oil, 20 g of lutein/zeaxanthin oil, 11.8 g of triglyceride, 2 g of mix tocopherol, and 10 g of decaglycerin monolaurate, and mixing and dissolving the resultant. Subsequently, 10 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 23.

[1-24] Preparation of Emulsion Composition According to Example 24

An oil phase was prepared by adding 32 g of 10% Haematococcus algae pigment oil, 20 g of lutein/zeaxanthin oil, 16.8 g of triglyceride, 2 g of mix tocopherol, and 5 g of decaglycerin monolaurate, and mixing and dissolving the resultant. Subsequently, 10 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 24. shows the formulations of the above emulsion compositions according to Examples 20 to 24.

[Table 4]

| Raw material | Example 20 Weight | Example 21 Weight | Example 22 Weight | Example 23 Weight | Example 24 Weight |
|---|---|---|---|---|---|
| 20% Haematococcus algae pigment oil (AstaReal Oil 200SS (astaxanthins content: 20.3%, carotenoids content, excluding astaxanthins: 3.2%, acyl glycerin content: 59.5%)) | — | — | — | — | — |
| 10% Haematococcus algae pigment oil (AstaReal L10 (astaxanthins content: 11.3%, carotenoids content, excluding astaxanthins: 7.5%, of acyl glycerin content: 70.2%)) | 32 | 32 | 32 | 32 | 32 |
| Lutein/zeaxanthin (Lutemax 2020 (produced by OmniActive Health Technologies)) | 20 | 20 | 20 | 20 | 20 |
| Medium chain fatty acid triglyceride | 6.8 | 10.8 | 14.8 | 11.8 | 16.8 |
| Mix tocopherol | 2 | 2 | 2 | 2 | 2 |
| Lecithin paste (lecithin content: 60% or more) | — | — | — | — | — |
| Tetraglycerin monooleate (SY-Glyster FMO-3S (produced by Sakamoto Yakuhin Kogyo Co., Ltd.)) | — | — | — | — | — |
| Decaglycerin monolaurate (NIKKOL DECAGLYN 1-L (produced by Nikko Chemicals Co., Ltd.)) | 15 | 15 | 15 | 10 | 5 |
| Sucrose stearate (HLB19) (DK ESTER SS (produced by DKS Co. Ltd.)) | — | — | — | — | — |
| Sucrose stearate (HLB5) (RYOTO Sugar Ester S-570 (produced by Mitsubishi-Kagaku Foods Corp.)) | — | — | — | — | — |
| Sucrose laurate (HLB16) ("RYOTO Sugar Ester L-1695 (produced by Mitsubishi-Kagaku Foods Corp.)) | 10 | 6 | 2 | 10 | 10 |
| Sucrose laurate (HLB1) "RYOTO Sugar Ester L-195 (produced by Mitsubishi-Kagaku Foods Corp.)) | — | — | — | — | — |
| Purified water | 5.2 | 5.2 | 5.2 | 5.2 | 5.2 |
| Ethanol | — | — | — | — | — |
| Propylene glycol | 6 | 6 | 6 | 6 | 6 |
| Glycerin | 3 | 3 | 3 | 3 | 3 |
| Total (g) | 100 | 100 | 100 | 100 | 100 |

[1-25] Preparation of Emulsion Composition According to Example 25

An oil phase was prepared by adding 32 g of 10% Haematococcus algae pigment oil, 20 g of lutein/zeaxanthin oil, 21.8 g of triglyceride, and 2 g of mix tocopherol, and mixing and dissolving the resultant. Subsequently, 5 g of sucrose stearate having an HLB value of 19, 5 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 25.

[1-26] Preparation of Emulsion Composition According to Example 26

An oil phase was prepared by adding 32 g of 10% Haematococcus algae pigment oil, 20 g of lutein/zeaxanthin oil, 11.8 g of triglyceride, 2 g of mix tocopherol, and 10.0 g of sucrose laurate having an HLB value of 1, and mixing and dissolving the resultant. Subsequently, 10.0 g of sucrose stearate having an HLB value of 19, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 26.

[1-27] Preparation of Emulsion Composition According to Example 27

An oil phase was prepared by adding 32 g of 10% Haematococcus algae pigment oil, 20 g of lutein/zeaxanthin oil, 11.8 g of triglyceride, 2 g of mix tocopherol, and 10.0 g of sucrose stearate having an HLB value of 5, and mixing and dissolving the resultant. Subsequently, 10 g of sucrose laurate having an HLB value of 16, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 27. shows the formulations of the above emulsion compositions according to Examples 25 to 27.

[Table 5]

| Raw material | Example 25 Weight | Example 26 Weight | Example 27 Weight |
|---|---|---|---|
| 20% Haematococcus algae pigment oil (AstaReal Oil 200SS (astaxanthins content: 20.3%, carotenoids content, excluding astaxanthins: 3.2%, acyl glycerin content: 59.5%)) | — | — | — |
| 10% Haematococcus algae pigment oil (AstaReal L10 (astaxanthins content: 11.3%, carotenoids content, excluding astaxanthins: 7.5%, of acyl glycerin content: 70.2%)) | 32 | 32 | 32 |
| Lutein/zeaxanthin (Lutemax 2020 (produced by OmniActive Health Technologies)) | 20 | 20 | 20 |
| Medium chain fatty acid triglyceride | 21.8 | 11.8 | 11.8 |
| Mix tocopherol | 2 | 2 | 2 |
| Lecithin paste (lecithin content: 60% or more) | — | — | — |
| Tetraglycerin monooleate (SY-Glyster FMO-3S (produced by Sakamoto Yakuhin Kogyo Co., Ltd.)) | — | — | — |
| Decaglycerin monolaurate (NIKKOL DECAGLYN 1-L (produced by Nikko Chemicals Co., Ltd.)) | — | — | — |
| Sucrose stearate (HLB19) (DK ESTER SS (produced by DKS Co. Ltd.)) | 5 | 10 | — |
| Sucrose stearate (HLB5) (RYOTO Sugar Ester S-570 (produced by Mitsubishi-Kagaku Foods Corp.)) | — | — | 10 |
| Sucrose laurate (HLB16) ("RYOTO Sugar Ester L-1695 (produced by Mitsubishi-Kagaku Foods Corp.)) | 5 | — | 10 |
| Sucrose laurate (HLB1) "RYOTO Sugar Ester L-195 (produced by Mitsubishi-Kagaku Foods Corp.)) | — | 10 | — |
| Purified water | 5.2 | 5.2 | 5.2 |
| Ethanol | — | — | — |
| Propylene glycol | 6 | 6 | 6 |
| Glycerin | 3 | 3 | 3 |
| Total (g) | 100 | 100 | 100 |

[1-28] Preparation of Emulsion Composition According to Example 28

An oil phase was prepared by adding 32 g of 10% Haematococcus algae pigment oil, 20 g of lutein/zeaxanthin oil, 6.8 g of triglyceride, 2 g of mix tocopherol, and 15 g of decaglycerin monolaurate, and mixing and dissolving the resultant. Subsequently, 10 g of sucrose stearate having an HLB value of 19, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 28.

[1-29] Preparation of Emulsion Composition According to

Example 29

An oil phase was prepared by adding 32 g of 10% Haematococcus algae pigment oil, 20 g of lutein/zeaxanthin oil, 10.8 g of triglyceride, 2 g of mix tocopherol, and 15 g of decaglycerin monolaurate, and mixing and dissolving the resultant. Subsequently, 6 g of sucrose stearate having an HLB value of 19, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 29.

[1-30] Preparation of Emulsion Composition According to Example 30

An oil phase was prepared by adding 32 g of 10% Haematococcus algae pigment oil, 20 g of lutein/zeaxanthin oil, 14.8 g of triglyceride, 2 g of mix tocopherol, and 15 g of decaglycerin monolaurate, and mixing and dissolving the resultant. Subsequently, 2 g of sucrose stearate having an HLB value of 19, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 30.

[1-31] Preparation of Emulsion Composition According to Example 31

An oil phase was prepared by adding 32 g of 10% Haematococcus algae pigment oil, 20 g of lutein/zeaxanthin oil, 11.8 g of triglyceride, 2 g of mix tocopherol, and 10 g of decaglycerin monolaurate, and mixing and dissolving the resultant. Subsequently, 10 g of sucrose stearate having an HLB value of 19, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 31.

[1-32] Preparation of Emulsion Composition According to Example 32

An oil phase was prepared by adding 32 g of 10% Haematococcus algae pigment oil, 20 g of lutein/zeaxanthin oil, 16.8 g of triglyceride, 2 g of mix tocopherol, and 5 g of decaglycerin monolaurate, and mixing and dissolving the resultant. Subsequently, 10 g of sucrose stearate having an HLB value of 19, 6 g of propylene glycol, and 3 g of glycerin were added to 5.2 g of purified water and dissolved to prepare an aqueous phase, and the oil phase and the aqueous phase were then mixed together to obtain an emulsion composition, which was used as an emulsion composition according to Example 32. shows the formulations of the above emulsion compositions according to Examples 28 to 32.

[Table 6]

| Raw material | Example 28 Weight | Example 29 Weight | Example 30 Weight | Example 31 Weight | Example 32 Weight |
|---|---|---|---|---|---|
| 20% Haematococcus algae pigment oil (AstaReal Oil 200SS (astaxanthins content: 20.3%, carotenoids content, excluding astaxanthins: 3.2%, acyl glycerin content: 59.5%)) | — | — | — | — | — |
| 10% Haematococcus algae pigment oil (AstaReal L10 (astaxanthins content: 11.3%, carotenoids content, excluding astaxanthins: 7.5%, of acyl glycerin content: 70.2%)) | 32 | 32 | 32 | 32 | 32 |
| Lutein/zeaxanthin (Lutemax 2020 (produced by OmniActive Health Technologies)) | 20 | 20 | 20 | 20 | 20 |
| Medium chain fatty acid triglyceride | 6.8 | 10.8 | 14.8 | 11.8 | 16.8 |
| Mix tocopherol | 2 | 2 | 2 | 2 | 2 |
| Lecithin paste (lecithin content: 60% or more) | — | — | — | — | — |
| Tetraglycerin monooleate (SY-Glyster FMO-3S (produced by Sakamoto Yakuhin Kogyo Co., Ltd.)) | — | — | — | — | — |
| Decaglycerin monolaurate (NIKKOL DECAGLYN 1-L (produced by Nikko Chemicals Co., Ltd.)) | 15 | 15 | 15 | 10 | 5 |
| Sucrose stearate (HLB19) (DK ESTER SS (produced by DKS Co. Ltd.)) | 10 | 6 | 2 | 10 | 10 |
| Sucrose stearate (HLB5) (RYOTO Sugar Ester S-570 (produced by Mitsubishi-Kagaku Foods Corp.)) | — | — | — | — | — |
| Sucrose laurate (HLB16) ("RYOTO Sugar Ester L-1695 (produced by Mitsubishi-Kagaku Foods Corp.)) | — | — | — | — | — |
| Sucrose laurate (HLB1) "RYOTO Sugar Ester L-195 (produced by Mitsubishi-Kagaku Foods Corp.)) | — | — | — | — | — |
| Purified water | 5.2 | 5.2 | 5.2 | 5.2 | 5.2 |
| Ethanol | — | — | — | — | — |
| Propylene glycol | 6 | 6 | 6 | 6 | 6 |
| Glycerin | 3 | 3 | 3 | 3 | 3 |
| Total (g) | 100 | 100 | 100 | 100 | 100 |

[1-33] Preparation of Emulsion Composition According to Example 33

An oil phase was prepared by adding 21.5 g of 20% Haematococcus algae pigment oil, 37.5 g of triglyceride, 2 g of mix tocopherol, 20 g of tetraglycerin monooleate, 6 g of decaglycerin monolaurate, and 0.4 g of ethanol, and mixing and dissolving the resultant. Subsequently, 1.52 g of sucrose laurate and 9 g of glycerin were added to 2.08 g of purified water and dissolved to prepare an aqueous phase, and the oil phase and the aqueous phase were then mixed together to obtain an emulsion composition, which was used as an emulsion composition according to Example 33.

[1-34] Preparation of Emulsion Composition According to Example 34

An oil phase was prepared by adding 43 g of 10% Haematococcus algae pigment oil, 12 g of triglyceride, 2 g of mix tocopherol, 18 g of tetraglycerin monooleate, and 1 g of ethanol, and mixing and dissolving the resultant. Subsequently, 6 g of sucrose stearate having an HLB value of 19, 3.8 g of sucrose laurate having an HLB value of 16, and 9 g of glycerin were added to 5.2 g of purified water and dissolved to prepare an aqueous phase, and the oil phase and the aqueous phase were then mixed together to obtain an emulsion composition, which was used as an emulsion composition according to Example 34.

[1-35] Preparation of Emulsion Composition According to Example 35

An oil phase was prepared by adding 43 g of 10% Haematococcus algae pigment oil, 11.8 g of triglyceride, 2 g of mix tocopherol, 18 g of tetraglycerin monooleate, 3 g of decaglycerin monolaurate, and 1 g of ethanol, and mixing and dissolving the resultant. Subsequently, 7 g of sucrose stearate having an HLB value of 19 and 9 g of glycerin were added to 5.2 g of purified water and dissolved to prepare an aqueous phase, and the oil phase and the aqueous phase were then mixed together to obtain an emulsion composition, which was used as an emulsion composition according to Example 35. shows the formulations of the above emulsion compositions according to Examples 33 to 35.

[Table 7]

| Raw material | Example 33 Weight | Example 34 Weight | Example 35 Weight |
|---|---|---|---|
| 20% Haematococcus algae pigment oil (AstaReal Oil 200SS (astaxanthins content: 20.3%, carotenoids content, excluding astaxanthins: 3.2%, acyl glycerin content: 59.5%)) | 21.5 | — | — |
| 10% Haematococcus algae pigment oil (AstaReal L10 (astaxanthins content: 11.3%, carotenoids content, excluding astaxanthins: 7.5%, of acyl glycerin content: 70.2%)) | — | 43 | 43 |
| Lutein/zeaxanthin (Lutemax 2020 (produced by OmniActive Health Technologies)) | — | — | — |
| Medium chain fatty acid triglyceride | 37.5 | 12 | 11.8 |
| Mix tocopherol | 2 | 2 | 2 |
| Lecithin paste (lecithin content: 60% or more) | — | — | — |
| Tetraglycerin monooleate (SY-Glyster FMO-3S (produced by Sakamoto Yakuhin Kogyo Co., Ltd.)) | 20 | 18 | 18 |
| Decaglycerin monolaurate (NIKKOL DECAGLYN 1-L (produced by Nikko Chemicals Co., Ltd.)) | 6 | — | 3 |
| Sucrose stearate (HLB19) (DK ESTER SS (produced by DKS Co. Ltd.)) | — | 6 | 7 |
| Sucrose stearate (HLB5) (RYOTO Sugar Ester S-570 (produced by Mitsubishi-Kagaku Foods Corp.)) | — | — | — |
| Sucrose laurate (HLB16) ("RYOTO Sugar Ester L-1695 (produced by Mitsubishi-Kagaku Foods Corp.)) | 1.52 | 3.8 | - |
| Sucrose laurate (HLB1) "RYOTO Sugar Ester L-195 (produced by Mitsubishi-Kagaku Foods Corp.)) | — | — | — |
| Purified water | 2.08 | 5.2 | 5.2 |
| Ethanol | 0.4 | 1 | 1 |
| Propylene glycol | — | — | — |
| Glycerin | 9 | 9 | 9 |
| Total (g) | 100 | 100 | 100 |

[1-36] Preparation of Emulsion Composition According to Example 36

An oil phase was prepared by adding 21.5 g of 20% Haematococcus algae pigment oil, 33.5 g of triglyceride, 2 g of mix tocopherol, 15 g of tetraglycerin monooleate, and 3 g of decaglycerin monolaurate, and mixing and dissolving the resultant. Subsequently, 6 g of sucrose stearate having an HLB value of 19, 3.8 g of sucrose laurate having an HLB value of 16, and 9 g of glycerin were added to 5.2 g of purified water and dissolved to prepare an aqueous phase, and the oil phase, the aqueous phase, and 1 g of ethanol were then mixed together to obtain an emulsion composition, which was used as an emulsion composition according to Example 36.

[1-37] Preparation of Emulsion Composition According to Example 37

An oil phase was prepared by adding 43 g of 10% Haematococcus algae pigment oil, 12 g of triglyceride, 2 g of mix tocopherol, 15 g of tetraglycerin monooleate, and 4.5 g of decaglycerin monolaurate, and mixing and dissolving the resultant. Subsequently, 4.5 g of sucrose stearate having an HLB value of 19, 3.8 g of sucrose laurate having an HLB value of 16, and 9 g of glycerin were added to 5.2 g of purified water and dissolved to prepare an aqueous phase, and the oil phase, the aqueous phase, and 1 g of ethanol were then mixed together to obtain an emulsion composition, which was used as an emulsion composition according to Example 37.

[1-38] Preparation of Emulsion Composition According to Example 38

An oil phase was prepared by adding 43 g of 10% Haematococcus algae pigment oil, 12 g of triglyceride, 2 g of mix tocopherol, 15 g of tetraglycerin monooleate, and 6 g of decaglycerin monolaurate, and mixing and dissolving the resultant. Subsequently, 3 g of sucrose stearate having an HLB value of 19, 3.8 g of sucrose laurate having an HLB value of 16, and 9 g of glycerin were added to 5.2 g of purified water and dissolved to prepare an aqueous phase, and the oil phase, the aqueous phase, and 1 g of ethanol were then mixed together to obtain an emulsion composition, which was used as an emulsion composition according to Example 38.

[1-39] Preparation of Emulsion Composition According to Example 39

An oil phase was prepared by adding 43 g of 10% Haematococcus algae pigment oil, 12 g of triglyceride, 2 g of mix tocopherol, 16 g of tetraglycerin monooleate, and 2 g of decaglycerin monolaurate, and mixing and dissolving the resultant. Subsequently, 6 g of sucrose stearate having an HLB value of 19, 3.8 g of sucrose laurate having an HLB value of 16, and 9 g of glycerin were added to 5.2 g of purified water and dissolved to prepare an aqueous phase, and the oil phase, the aqueous phase, and 1 g of ethanol were then mixed together to obtain an emulsion composition, which was used as an emulsion composition according to Example 39.

[1-40] Preparation of Emulsion Composition According to Example 40

An oil phase was prepared by adding 43 g of 10% Haematococcus algae pigment oil, 12 g of triglyceride, 2 g of mix tocopherol, 17.5 g of tetraglycerin monooleate, and 0.5 g of decaglycerin monolaurate, and mixing and dissolving the resultant. Subsequently, 6 g of sucrose stearate having an HLB value of 19, 3.8 g of sucrose laurate having an HLB value of 16, and 9 g of glycerin were added to 5.2 g of purified water and dissolved to prepare an aqueous phase, and the oil phase, the aqueous phase, and 1 g of ethanol were then mixed together to obtain an emulsion composition, which was used as an emulsion composition according to Example 40.

[1-41] Preparation of Emulsion Composition According to Example 41

An oil phase was prepared by adding 21.5 g of lutein/zeaxanthin, 33.5 g of triglyceride, 2 g of mix tocopherol, 15 g of tetraglycerin monooleate, and 3 g of decaglycerin monolaurate, and mixing and dissolving the resultant. Subsequently, 6 g of sucrose stearate having an HLB value of 19, 3.8 g of sucrose laurate having an HLB value of 16, and 9 g of glycerin were added to 5.2 g of purified water and dissolved to prepare an aqueous phase, and the oil phase, the aqueous phase, and 1 g of ethanol were then mixed together to obtain an emulsion composition, which was used as an emulsion composition according to Example 41.

[1-42] Preparation of Emulsion Composition According to Example 42

An oil phase was prepared by adding 21.5 g of 20% Haematococcus algae pigment oil, 21.5 g of lutein/zeaxanthin, 12 g of triglyceride, 2 g of mix tocopherol, 15 g of tetraglycerin monooleate, and 3 g of decaglycerin monolaurate, and mixing and dissolving the resultant. Subsequently, 6 g of sucrose stearate having an HLB value of 19, 3.8 g of sucrose laurate having an HLB value of 16, and 9 g of glycerin were added to 5.2 g of purified water and dissolved to prepare an aqueous phase, and the oil phase, the aqueous phase, and 1 g of ethanol were then mixed together to obtain an emulsion composition, which was used as an emulsion composition according to Example 42. shows the formulations of the above emulsion compositions according to Examples 36 to 42.

[Table 8]

| Raw material | Example 36 Weight | Example 37 Weight | Example 38 Weight | Example 39 Weight | Example 40 Weight | Example 41 Weight | Example 42 Weight |
|---|---|---|---|---|---|---|---|
| 20% Haematococcus algae pigment oil (AstaReal Oil 200SS (astaxanthins content: 20.3%, carotenoids content, excluding astaxanthins: 3.2%, acyl glycerin content: 59.5%)) | 21.5 | — | — | — | — | — | 21.5 |
| 10% Haematococcus algae pigment oil (AstaReal L10 (astaxanthins content: 11.3%, carotenoids content, excluding astaxanthins: 7.5%, of acyl glycerin content: 70.2%)) | | 43 | 43 | 43 | 43 | — | — |
| Lutein/zeaxanthin (Lutemax 2020 (produced by OmniActive Health Technologies)) | — | — | — | — | — | 21.5 | 21.5 |
| Medium chain fatty acid triglyceride | 33.5 | 12 | 12 | 12 | 12 | 33.5 | 12 |
| Mix tocopherol | 2 | 2 | 2 | 2 | 2 | 2 | 2 |
| Lecithin paste (lecithin content: 60% or more) | — | — | — | — | — | — | — |
| Tetraglycerin monooleate (SY-Glyster FMO-3S (produced by Sakamoto Yakuhin Kogyo Co., Ltd.)) | 15 | 15 | 15 | 16 | 17.5 | 15 | 15 |
| Decaglycerin monolaurate (NIKKOL DECAGLYN 1-L (produced by Nikko Chemicals Co., Ltd.)) | 3 | 4.5 | 6 | 2 | 0.5 | 3 | 3 |
| Sucrose stearate (HLB19) (DK ESTER SS (produced by DKS Co. Ltd.)) | 6 | 4.5 | 3 | 6 | 6 | 6 | 6 |
| Sucrose stearate (HLB5) (RYOTO Sugar Ester S-570 (produced by Mitsubishi-Kagaku Foods Corp.)) | — | — | — | — | — | — | — |
| Sucrose laurate (HLB16) ("RYOTO Sugar Ester L-1695 (produced by Mitsubishi-Kagaku Foods Corp.)) | 3.8 | 3.8 | 3.8 | 3.8 | 3.8 | 3.8 | 3.8 |
| Sucrose laurate (HLB1) "RYOTO Sugar Ester L-195 (produced by Mitsubishi-Kagaku Foods Corp.)) | — | — | — | — | — | — | — |
| Purified water | 5.2 | 5.2 | 5.2 | 5.2 | 5.2 | 5.2 | 5.2 |
| Ethanol | 1 | 1 | 1 | 1 | 1 | 1 | 1 |
| Propylene glycol | — | — | — | — | — | — | — |
| Glycerin | 9 | 9 | 9 | 9 | 9 | 9 | 9 |
| Total (g) | 100 | 100 | 100 | 100 | 100 | 100 | 100 |

2. Preparation of Comparative Compositions

Next, comparative compositions 1 to 4 were prepared as described below. In preparation of comparative compositions 1 to 4, "AstaReal Oil 200SS (produced by AstaReal Co., Ltd.)" was used as "20% Haematococcus algae pigment oil, "AstaReal L10 (produced by AstaReal Co., Ltd.)" was used as "10% Haematococcus algae pigment oil", and "Lutemax 2020 (produced by OmniActive Health Technologies)" was used as "lutein/zeaxanthin".
Used as comparative compositions 1, 2, and 3, were 20% Haematococcus algae pigment oil, 10% Haematococcus algae pigment oil, and lutein/zeaxanthin oil, respectively. Comparative composition 4 was prepared by mixing 62 g of 10% Haematococcus algae pigment oil and 38 g of lutein/zeaxanthin oil. shows the formulations of comparative compositions 1 to 4.

[Table 9]

| Raw material | Comparative composition 1 Weight | Comparative composition 2 Weight | Comparative composition 3 Weight | Comparative composition 4 Weight |
|---|---|---|---|---|
| 20% Haematococcus algae pigment oil (AstaReal Oil 200SS (astaxanthins content: 20.3%, carotenoids content, excluding astaxanthins: 3.2%, acyl glycerin content: 59.5%)) | 100 | — | — | — |
| 10% Haematococcus algae pigment oil (AstaReal L10 (astaxanthins content: 11.3%, carotenoids content, excluding astaxanthins: 7.5%, of acyl glycerin content: 70.2%)) | — | 100 | — | 62 |
| Lutein/zeaxanthin (Lutemax 2020 (produced by OmniActive Health Technologies)) | — | — | 100 | 38 |
| Total (g) | 100 | 100 | 100 | 100 |

3. Evaluation of Affinity with Soft Capsule Shell

Affinity with a soft capsule shell was evaluated for the emulsion compositions according to Examples 1 to 42 through the following procedure.

A plant-based coated MCT soft capsule was precipitated in each of the emulsion compositions according to Examples 1 to 42 and left to stand at room temperature for 2 days, and thereafter the soft capsule was taken out. The shell of each soft capsule was checked for deformation and softening, and when no deformation, depression, or softening of the soft capsule shell occurred, the emulsion composition was considered to have low affinity with the soft capsule shell (O), and when deformation, depression, or softening of the soft capsule shell occurred, the emulsion composition was considered to have high affinity with the soft capsule shell (×). Figure 1 shows the results.

As demonstrated in Figure 1, the deformation, depression, or softening of the soft capsule shell did not occur for all the emulsion compositions according to Examples 1 to 42, indicating low affinity with the soft capsule shell (○), and this result revealed that the emulsion composition according to the present invention can be formulated into a soft capsule formulation.

4. Systemic Absorption Test

The emulsion compositions according to Examples 1 to 42 and comparative compositions 1 to 4 prepared were each subjected to a systemic absorption test for carotenoids through the following procedure.

[3-1] Preparation of Samples

To Wistar rats fasted overnight (male, 4 to 6 animals per group, body weight: 200 to 250 g, 8- to 10-week-old), the emulsion compositions according to Examples 1 to 42 and comparative compositions 1 to 4 were administered with a feeding needle. At the administration, the emulsion compositions according to Examples 1 to 42 and comparative compositions 1 to 4 were each diluted with medium chain fatty acid triglyceride to prepare for administration of 4.4 cc/kg rat weight, so that when the composition to be administered contained only astaxanthin as a carotenoid, the dose of astaxanthin (in terms of the free form) reached 100 mg/kg rat weight, when the composition to be administered contained only lutein/zeaxanthin, the dose of lutein reached 100 mg/kg rat weight and the dose of zeaxanthin reached 20 mg/kg rat weight, and when the composition to be administered contained astaxanthin and lutein/zeaxanthin, the dose of astaxanthin (in terms of the free form) reached 100 mg/kg rat weight and the dose of lutein reached 120 mg/kg rat weight and the dose of zeaxanthin reached 25 mg/kg rat weight. After the administration, and 3, 6, 9, and 24 hours after the administration, blood was collected from the jugular vein, and the plasma was separated. The plasma obtained was stored at -80°C before quantitative analysis of carotenoids.

To 100 $\mu$L of each plasma sample obtained, 500 $\mu$L of ethanol solution of butylated hydroxytoluene (BHT; 50 $\mu$g/mL) and 100 $\mu$L of 100 ng/mL acetone solution of an internal standard (trans-$\beta$-apo-8'-carotenal, 10829; produced by Sigma-Aldrich Co. LLC) were added, the sample was vigorously stirred by using a vortex mixer for 15 seconds, 5 mL of hexane was then added thereto, and the sample was further vigorously stirred by using a vortex mixer for 15 seconds. This stirring process was performed three times, and centrifugation was then performed at a rotational frequency of 3500 rpm for 10 minutes. After the centrifugation, 4 mL of the resulting supernatant was collected, and filtered through a membrane filter with a mesh size of 0.45 $\mu$m. The resulting filtrate was concentrated with a centrifugal evaporator, and then redissolved in 150 $\mu$L of acetone to obtain a sample, which was subjected to reverse-phase HPLC.

Carotenoid standard solutions were prepared as follows: 1 mL of 2 $\mu$g/mL acetone solution of astaxanthin standard reagent (460-031-M250; Astaxanthin, produced by Alexis Biochemicals) and 2.5 mL of 800 ng/mL acetone solution of an internal standard (trans-$\beta$-apo-8'-carotenal, 10829; produced by Sigma-Aldrich Co. LLC) were mixed, and the resulting solution was diluted with acetone to a volume of 20.0 mL in a graduated cylinder to prepare an astaxanthin standard solution; 2 mL of 2.67 $\mu$g/mL acetone solution of lutein standard and 2.5 mL of 800 ng/mL acetone solution of an internal standard (trans-$\beta$-apo-8'-carotenal, 10829; produced by Sigma-Aldrich Co. LLC) were mixed, and the resulting solution was diluted with acetone to a volume of 20.0 mL in a graduated cylinder to prepare a lutein standard solution; and 2 mL of 1.55 $\mu$g/mL acetone solution of zeaxanthin standard reagent and 2.5 mL of 800 ng/mL acetone solution of an internal standard (trans-$\beta$-apo-8'-carotenal, 10829; produced by Sigma-Aldrich Co. LLC) were mixed, and the resulting solution was diluted with acetone to a volume of 20.0 mL in a graduated cylinder to prepare a zeaxanthin standard solution. The standard solutions prepared were subjected to reverse-phase HPLC in the same manner, and astaxanthin, lutein, and zeaxanthin concentrations were determined through comparison of peak area ratios acquired.

[3-2] HPLC Conditions

HPLC was performed by using a Shimadzu LC20A Series (pump: LC-20AD, degasser: DGU-20A5R, autosampler: SIL-20AC, column oven: CTO-20AC, detector: SPD-20AV, system controller: CBM-20A) and a YMC-Carotenoid (4.6 × 250 mm, particle size: 5 $\mu$m) as an analysis column. For the mobile phase, solution A (methanol), solution B (tert-butyl methyl ether), and solution C (1%(v/v) aqueous solution of phosphoric acid) were used. Gradient elution was performed in the following manner: the mixing ratio between solution A and solution B was 81:13 (% ratio) at initiation; the ratio of solution B reached 28% after the lapse of 15 minutes, and 78% after the lapse of 27 minutes; the composition with the mixing ratio of solution B being 78% was retained until the lapse of 31 minutes; thereafter the mixing ratio was returned to that at initiation after the lapse of 31.01 minutes; and elution was performed at the composition at initiation until the lapse of 40 minutes. The mixing ratio of solution C in the composition was always retained at 6%. The temperature of the column oven was set to 25°C, and the detection wavelength of the UV/VIS detector was set to 470 nm for measurement, and the flow rate was 1 mL/min.

Calculated was area under the blood concentration-time curve after 0 to 24 hours ($AUC_{0-24hr}$; hereinafter, abbreviated as "AUC") for each of astaxanthin, lutein, and zeaxanthin in the systemic absorption test conducted for the emulsion compositions according to Examples 1 to 42 and comparative compositions 1 to 4. AUC ratios of the emulsion compositions according to Examples 1 to 42 to comparative compositions 1 to 4 were calculated by using the following expression, and Figure 2 shows the results.

AUC Ratio = AUC value of any of emulsion compositions according to Examples 1 to 42 / AUC value of corresponding composition among comparative compositions 1 to 4

As shown in Figure 2, the AUC ratios of the emulsion compositions according to Examples 1 to 40 and 42 to the corresponding comparative composition for astaxanthin were 2.7, 1.5, 2.4, 1.7, 1.5, 1.3, 1.9, 1.2, 1.9, 1.2, 2.3, 1.1, 1.2, 2.0, 1.3, 1.8, 1.2, 1.1, 1.5, 1.6, 1.9, 1.4, 2.1, 1.7, 1.3, 1.2, 1.6, 2.1, 1.8, 1.4, 2.2, 2.1, 1.8, 4.6, 2.0, 1.5, 2.6, 1.8, 2.6, 2.2, and 1.8, respectively. The AUC ratios of the emulsion compositions according to Examples 2 to 10, 12 to 15, 17 to 32, 41, and 42 to the corresponding comparative composition for lutein were 1.4, 1.7, 1.3, 1.6, 1.2, 1.3, 1.3, 1.4, 1.2, 1.0, 1.1, 1.3, 1.1, 1.4, 1.5, 1.0, 1.1, 1.4, 1.3, 1.7, 1.3, 1.3, 0.9, 1.2, 1.2, 1.1, 1.0, 1.2, 1.2, 1.3, and 1.3, respectively. The AUC ratios of the emulsion compositions according to Examples 2 to 10, 12 to 15, 17 to 32, 41, and 42 to the corresponding comparative composition for zeaxanthin were 2.5, 10.1, 9.6, 10.8, 2.8, 4.1, 3.3, 4.7, 3.7, 3.6, 5.4, 3.6, 4.9, 1.4, 1.9, 0.6, 2.0, 4.0, 3.4, 3.7, 5.0, 5.5, 2.6, 2.5, 1.4, 0.8, 0.8, 1.9, 1.5, 13.3, and 1.8.

From these results, showing that at least any value of the AUC ratios of each of the emulsion compositions according to Examples 1 to 42 and the corresponding comparative composition for astaxanthin, lutein, and zeaxanthin was 1.4 or higher (among the AUC ratios of each of the emulsion compositions according to Examples 1 to 42 to the corresponding comparative composition for astaxanthin, lutein, and zeaxanthin, the highest value was 1.4 or higher), the emulsion composition according to the present invention was revealed to allow higher systemic absorption for carotenoids than conventional emulsion compositions.

5. Evaluation of Homogeneity

Homogeneity was evaluated for the emulsion compositions according to Examples 11, 34, and 36 through the following procedure.

The emulsion compositions according to Examples 11, 34, and 36 with the loading of ethanol set to 0 g (0% by weight), 0.1 g (0.1% by weight), 0.25 g (0.25% by weight), 0.5 g (0.5% by weight), 0.75 g (0.75% by weight), or 1 g (1% by weight) were prepared, where the total amount was adjusted with the loading of triglyceride. The emulsion composition according to Example 36 was prepared with addition of a dihydric alcohol such as propylene glycol or a (polyhydric) sugar alcohol such as xylitol, sorbitol, lactitol, and erythritol in place of ethanol (monohydric alcohol). Each of the emulsion compositions prepared were heated at 60°C for 20 minutes and then sieved through an 80-mesh stainless steel test sieve, and the weight (g) of the test sieve after sieving was measured. Subsequently, the weight (g) of the test sieve before sieving was subtracted from the weight (g) of the test sieve measured after sieving to calculate the weight (g) of residues of each of the emulsion compositions according to Examples 11, 34, and 36 on the test sieve after sieving, and the weight of residues on the test sieve after sieving was then divided by the amount of the emulsion composition subjected to sieving to calculate the residue proportion (% by weight) on the test sieve after sieving. Then, when the calculated residue proportion (% by weight) on the test sieve after sieving was lower than 10%, the emulsion composition was considered to be highly homogeneous (O), and when the residue proportion was 10% or higher and lower than 20%, the emulsion composition was considered to have common homogeneity (△), and when the residue proportion was 30% or higher, the emulsion composition was considered to be inhomogeneous (×). Figures 3 to 6 show the formulations of the emulsion compositions prepared and the evaluation results.

As shown in Figures 3 to 5, it was demonstrated that emulsion compositions prepared without addition of ethanol were not homogeneous, and emulsion compositions prepared with addition of ethanol had homogeneity. In addition, it was demonstrated that emulsion compositions prepared with ethanol in a loading of 0.5% by weight or 0.75% by weight or more were highly homogeneous. As shown in Figure 6, it was demonstrated that emulsion compositions prepared even with addition of propylene glycol (dihydric alcohol) or a (polyhydric) sugar alcohol such as xylitol, sorbitol, lactitol, and erythritol in place of ethanol (monohydric alcohol) had homogeneity similar to those with addition of ethanol. These results revealed that the emulsion composition according to the present invention had homogeneity by virtue of inclusion of an alcohol.

5. Soft Capsule

A carotenoid-containing soft capsule is obtained in such a manner that 55 g of any of the emulsion compositions produced in Examples 1 to 42, 40 g of diglycerin laurate (RIKEMAL L-71-D; produced by RIKEN VITAMIN CO., LTD.), and 5 g of polyoxyethylenesorbitan fatty acid ester were mixed to a homogeneous state, and a soft capsule is filled with the mixture by using a filling machine.

**Claims**

1. An emulsion composition comprising a carotenoid and an emulsifier, the emulsifier being any combination of the following (a), (b), (c), (d), (e), (f), (g), (h), (i), and (j):

   (a) tetraglycerin monooleate and decaglycerin monolaurate;
   (b) tetraglycerin monooleate and sucrose stearate;
   (c) tetraglycerin monooleate and sucrose laurate;
   (d) decaglycerin monolaurate and sucrose laurate;
   (e) sucrose stearate and sucrose laurate;
   (f) tetraglycerin monooleate, decaglycerin monolaurate, and sucrose stearate;
   (g) tetraglycerin monooleate, decaglycerin monolaurate, and sucrose laurate;
   (h) tetraglycerin monooleate, sucrose stearate, and sucrose laurate;
   (i) decaglycerin monolaurate, sucrose stearate, and sucrose laurate; and
   (j) tetraglycerin monooleate, decaglycerin monolaurate, sucrose stearate, and sucrose laurate.

2. An emulsion composition comprising a carotenoid, and decaglycerin monolaurate and sucrose stearate, and being free of lecithin.

3. The emulsion composition according to claim 1 or 2, comprising an alcohol.

4. The emulsion composition according to any one of claims 1 to 3, wherein the carotenoid is one or more selected from the group consisting of lutein, zeaxanthin, astaxanthin, lycopene, β-carotene, γ-carotene, phytofluene, phytoene, canthaxanthin, β-cryptoxanthin, capsanthin, fucoxanthin, and fatty acid esters thereof.

5. The emulsion composition according to claim 4, wherein the astaxanthin is derived from a Haematococcus algae extract.

6. The emulsion composition according to any one of claims 1 to 5, comprising a mineral and/or a vitamin.

7. A soft capsule formulation comprising the emulsion composition according to any one of claims 1 to 6.

Fig. 1

| | Example 1 | Example 2 | Example 3 | Example 4 | Example 5 | Example 6 | Example 7 | Example 8 |
|---|---|---|---|---|---|---|---|---|
| Compatibility with soft capsule | O | O | O | O | O | O | O | O |

| | Example 9 | Example 10 | Example 11 | Example 12 | Example 13 | Example 14 | Example 15 | Example 16 |
|---|---|---|---|---|---|---|---|---|
| Compatibility with soft capsule | O | O | O | O | O | O | O | O |

| | Example 17 | Example 18 | Example 19 | Example 20 | Example 21 | Example 22 | Example 23 | Example 24 |
|---|---|---|---|---|---|---|---|---|
| Compatibility with soft capsule | O | O | O | O | O | O | O | O |

| | Example 25 | Example 26 | Example 27 | Example 28 | Example 29 | Example 30 | Example 31 | Example 32 |
|---|---|---|---|---|---|---|---|---|
| Compatibility with soft capsule | O | O | O | O | O | O | O | O |

| | Example 33 | Example 34 | Example 35 |
|---|---|---|---|
| Compatibility with soft capsule | O | O | O |

| | Example 36 | Example 37 | Example 38 | Example 39 | Example 40 | Example 41 | Example 42 |
|---|---|---|---|---|---|---|---|
| Compatibility with soft capsule | O | O | O | O | O | O | O |

Fig. 2

| | Example 1 | Example 2 | Example 3 | Example 4 | Example 5 | Example 6 | Example 7 | Example 8 |
|---|---|---|---|---|---|---|---|---|
| AUC ratio for astaxanthin | 2.7 | 1.5 | 2.4 | 1.7 | 1.5 | 1.3 | 1.9 | 1.2 |
| AUC ratio for lutein | — | 1.4 | 1.7 | 1.3 | 1.6 | 1.2 | 1.3 | 1.3 |
| AUC ratio for zeaxanthin | — | 2.5 | 10.1 | 9.6 | 10.8 | 2.8 | 4.1 | 3.3 |

| | Example 9 | Example 10 | Example 11 | Example 12 | Example 13 | Example 14 | Example 15 | Example 16 |
|---|---|---|---|---|---|---|---|---|
| AUC ratio for astaxanthin | 1.9 | 1.2 | 2.3 | 1.1 | 1.2 | 2.0 | 1.3 | 1.8 |
| AUC ratio for lutein | 1.4 | 1.2 | — | 1.0 | 1.1 | 1.3 | 1.1 | — |
| AUC ratio for zeaxanthin | 4.7 | 3.7 | — | 3.6 | 5.4 | 3.6 | 4.9 | — |

| | Example 17 | Example 18 | Example 19 | Example 20 | Example 21 | Example 22 | Example 23 | Example 24 |
|---|---|---|---|---|---|---|---|---|
| AUC ratio for astaxanthin | 1.2 | 1.1 | 1.5 | 1.6 | 1.9 | 1.4 | 2.1 | 1.7 |
| AUC ratio for lutein | 1.4 | 1.5 | 1.0 | 1.1 | 1.4 | 1.3 | 1.7 | 1.3 |
| AUC ratio for zeaxanthin | 1.4 | 1.9 | 0.6 | 2.0 | 4.0 | 3.4 | 3.7 | 5.0 |

| | Example 25 | Example 26 | Example 27 | Example 28 | Example 29 | Example 30 | Example 31 | Example 32 |
|---|---|---|---|---|---|---|---|---|
| AUC ratio for astaxanthin | 1.3 | 1.2 | 1.6 | 2.1 | 1.8 | 1.4 | 2.2 | 2.1 |
| AUC ratio for lutein | 1.3 | 0.9 | 1.2 | 1.2 | 1.1 | 1.0 | 1.2 | 1.2 |
| AUC ratio for zeaxanthin | 5.5 | 2.6 | 2.5 | 1.4 | 0.8 | 0.8 | 1.9 | 1.5 |

| | Example 33 | Example 34 | Example 35 |
|---|---|---|---|
| AUC ratio for astaxanthin | 1.8 | 4.6 | 2.0 |
| AUC ratio for lutein | — | — | — |
| AUC ratio for zeaxanthin | — | — | — |

| | Example 36 | Example 37 | Example 38 | Example 39 | Example 40 | Example 41 | Example 42 |
|---|---|---|---|---|---|---|---|
| AUC ratio for astaxanthin | 1.5 | 2.6 | 1.8 | 2.6 | 2.2 | — | 1.8 |
| AUC ratio for lutein | — | — | — | — | — | 1.3 | 1.3 |
| AUC ratio for zeaxanthin | — | — | — | — | — | 13.3 | 1.8 |

Fig. 3

| Raw material | Example 11 | | | | | |
|---|---|---|---|---|---|---|
| | Alcohol 1% | Alcohol 0.75% | Alcohol 0.5% | Alcohol 0.25% | Alcohol 0.1% | Alcohol 0% |
| 20% Haematococcus algae pigment oil (AstaReal Oil 200SS (astaxanthins content: 20.3%, carotenoids content, excluding astaxanthins: 3.2%, acyl glycerin content: 59.5%)) | — | — | — | — | — | — |
| 10% Haematococcus algae pigment oil (AstaReal L10 (astaxanthins content: 11.3%, carotenoids content, excluding astaxanthins: 7.5%, of acyl glycerin content: 70.2%)) | 43.00 | 43.00 | 43.00 | 43.00 | 43.00 | 43.00 |
| Lutein/zeaxanthin (Lutemax 2020 (produced by OmniActive Health Technologies)) | — | — | — | — | — | — |
| Medium chain fatty acid triglyceride | 12.00 | 12.25 | 12.50 | 12.75 | 12.90 | 13.00 |
| Mix tocopherol | 2.00 | 2.00 | 2.00 | 2.00 | 2.00 | 2.00 |
| Lecithin paste (lecithin content: 60% or more) | — | — | — | — | — | — |
| Tetraglycerin monooleate (SY-Glyster FMO-3S (produced by Sakamoto Yakuhin Kogyo Co., Ltd.)) | 18.00 | 18.00 | 18.00 | 18.00 | 18.00 | 18.00 |
| Decaglycerin monolaurate (NIKKOL DECAGLYN 1-L (produced by Nikko Chemicals Co., Ltd.)) | — | — | — | — | — | — |
| Sucrose stearate (HLB19) (DK ESTER SS (produced by DKS Co. Ltd.)) | 9.80 | 9.80 | 9.80 | 9.80 | 9.80 | 9.80 |
| Sucrose stearate (HLB5) (RYOTO Sugar Ester S-570 (produced by Mitsubishi-Kagaku Foods Corp.)) | — | — | — | — | — | — |
| Sucrose laurate (HLB16) ("RYOTO Sugar Ester L-1695 (produced by Mitsubishi-Kagaku Foods Corp.)) | — | — | — | — | — | — |
| Sucrose laurate (HLB1) "RYOTO Sugar Ester L-195 (produced by Mitsubishi-Kagaku Foods Corp.)) | — | — | — | — | — | — |
| Purified water | 5.20 | 5.20 | 5.20 | 5.20 | 5.20 | 5.20 |
| Ethanol | 1.00 | 0.75 | 0.50 | 0.25 | 0.10 | 0.00 |
| Propylene glycol | — | — | — | — | — | — |
| Sorbitol | — | — | — | — | — | — |
| Glycerin | 9.00 | 9.00 | 9.00 | 9.00 | 9.00 | 9.00 |
| Total (g) | 100.00 | 100.00 | 100.00 | 100.00 | 100.00 | 100.00 |
| Homogeneity of formulation (state of dispersion) | ○ | ○ | ○ | △ | △ | × |

Fig. 4

| | Example 34 | | | | | |
|---|---|---|---|---|---|---|
| Raw material | Alcohol 1% | Alcohol 0.75% | Alcohol 0.5% | Alcohol 0.25% | Alcohol 0.1% | Alcohol 0% |
| 20% Haematococcus algae pigment oil (AstaReal Oil 200SS (astaxanthins content: 20.3%, carotenoids content, excluding astaxanthins: 3.2%, acyl glycerin content: 59.5%)) | — | — | — | — | — | — |
| 10% Haematococcus algae pigment oil (AstaReal L10 (astaxanthins content: 11.3%, carotenoids content, excluding astaxanthins: 7.5%, of acyl glycerin content: 70.2%)) | 43.00 | 43.00 | 43.00 | 43.00 | 43.00 | 43.00 |
| Lutein/zeaxanthin (Lutemax 2020 (produced by OmniActive Health Technologies)) | — | — | — | — | — | — |
| Medium chain fatty acid triglyceride | 12.00 | 12.25 | 12.50 | 12.75 | 12.90 | 13.00 |
| Mix tocopherol | 2.00 | 2.00 | 2.00 | 2.00 | 2.00 | 2.00 |
| Lecithin paste (lecithin content: 60% or more) | — | — | — | — | — | — |
| Tetraglycerin monooleate (SY-Glyster FMO-3S (produced by Sakamoto Yakuhin Kogyo Co., Ltd.)) | 18.00 | 18.00 | 18.00 | 18.00 | 18.00 | 18.00 |
| Decaglycerin monolaurate (NIKKOL DECAGLYN 1-L (produced by Nikko Chemicals Co., Ltd.)) | — | — | — | — | — | — |
| Sucrose stearate (HLB19) (DK ESTER SS (produced by DKS Co. Ltd.)) | 6.00 | 6.00 | 6.00 | 6.00 | 6.00 | 6.00 |
| Sucrose stearate (HLB5) (RYOTO Sugar Ester S-570 (produced by Mitsubishi-Kagaku Foods Corp.)) | — | — | — | — | — | — |
| Sucrose laurate (HLB16) ("RYOTO Sugar Ester L-1695 (produced by Mitsubishi-Kagaku Foods Corp.)) | 3.80 | 3.80 | 3.80 | 3.80 | 3.80 | 3.80 |
| Sucrose laurate (HLB1) "RYOTO Sugar Ester L-195 (produced by Mitsubishi-Kagaku Foods Corp.)) | — | — | — | — | — | — |
| Purified water | 5.20 | 5.20 | 5.20 | 5.20 | 5.20 | 5.20 |
| Ethanol | 1.00 | 0.75 | 0.50 | 0.25 | 0.10 | 0.00 |
| Propylene glycol | — | — | — | — | — | — |
| Sorbitol | — | — | — | — | — | — |
| Glycerin | 9.00 | 9.00 | 9.00 | 9.00 | 9.00 | 9.00 |
| Total (g) | 100.00 | 100.00 | 100.00 | 100.00 | 100.00 | 100.00 |
| Homogeneity of formulation (state of dispersion) | O | O | △ | △ | △ | × |

Fig. 5

| | Example 36 | | | | | |
|---|---|---|---|---|---|---|
| Raw material | Alcohol 1% | Alcohol 0.75% | Alcohol 0.5% | Alcohol 0.25% | Alcohol 0.1% | Alcohol 0% |
| 20% Haematococcus algae pigment oil (AstaReal Oil 200SS (astaxanthins content: 20.3%, carotenoids content, excluding astaxanthins: 3.2%, acyl glycerin content: 59.5%)) | 21.50 | 21.50 | 21.50 | 21.50 | 21.50 | 21.50 |
| 10% Haematococcus algae pigment oil (AstaReal L10 (astaxanthins content: 11.3%, carotenoids content, excluding astaxanthins: 7.5%, of acyl glycerin content: 70.2%)) | — | — | — | — | — | — |
| Lutein/zeaxanthin (Lutemax 2020 (produced by OmniActive Health Technologies)) | — | — | — | — | — | — |
| Medium chain fatty acid triglyceride | 33.50 | 33.75 | 34.00 | 34.25 | 34.40 | 34.50 |
| Mix tocopherol | 2.00 | 2.00 | 2.00 | 2.00 | 2.00 | 2.00 |
| Lecithin paste (lecithin content: 60% or more) | — | — | — | — | — | — |
| Tetraglycerin monooleate (SY-Glyster FMO-3S (produced by Sakamoto Yakuhin Kogyo Co., Ltd.)) | 15.00 | 15.00 | 15.00 | 15.00 | 15.00 | 15.00 |
| Decaglycerin monolaurate (NIKKOL DECAGLYN 1-L (produced by Nikko Chemicals Co., Ltd.)) | 3.00 | 3.00 | 3.00 | 3.00 | 3.00 | 3.00 |
| Sucrose stearate (HLB19) (DK ESTER SS (produced by DKS Co. Ltd.)) | 6.00 | 6.00 | 6.00 | 6.00 | 6.00 | 6.00 |
| Sucrose stearate (HLB5) (RYOTO Sugar Ester S-570 (produced by Mitsubishi-Kagaku Foods Corp.)) | — | — | — | — | — | — |
| Sucrose laurate (HLB16) ("RYOTO Sugar Ester L-1695 (produced by Mitsubishi-Kagaku Foods Corp.)) | 3.80 | 3.80 | 3.80 | 3.80 | 3.80 | 3.80 |
| Sucrose laurate (HLB1) "RYOTO Sugar Ester L-195 (produced by Mitsubishi-Kagaku Foods Corp.)) | — | — | — | — | — | — |
| Purified water | 5.20 | 5.20 | 5.20 | 5.20 | 5.20 | 5.20 |
| Ethanol | 1.00 | 0.75 | 0.50 | 0.25 | 0.10 | 0.00 |
| Propylene glycol | — | — | — | — | — | — |
| Sorbitol | — | — | — | — | — | — |
| Glycerin | 9.00 | 9.00 | 9.00 | 9.00 | 9.00 | 9.00 |
| Total (g) | 100.00 | 100.00 | 100.00 | 100.00 | 100.00 | 100.00 |
| Homogeneity of formulation (state of dispersion) | ○ | ○ | △ | △ | △ | × |

Figure 6

| | Example 36 | | | | | |
|---|---|---|---|---|---|---|
| Raw material | Weight | | | | | |
| 20% Haematococcus algae pigment oil (AstaReal Oil 200SS (astaxanthins content: 20.3%, carotenoids content, excluding astaxanthins: 3.2%, acyl glycerin content: 59.5%)) | 21.50 | 21.50 | 21.50 | 21.50 | 21.50 | 21.50 |
| 10% Haematococcus algae pigment oil (AstaReal L10 (astaxanthins content: 11.3%, carotenoids content, excluding astaxanthins: 7.5%, of acyl glycerin content: 70.2%)) | — | — | — | — | — | — |
| Lutein/zeaxanthin (Lutemax 2020 (produced by OmniActive Health Technologies)) | — | — | — | — | — | — |
| Medium chain fatty acid triglyceride | 33.50 | 33.50 | 33.50 | 33.50 | 33.50 | 33.50 |
| Mix tocopherol | 2.00 | 2.00 | 2.00 | 2.00 | 2.00 | 2.00 |
| Lecithin paste (lecithin content: 60% or more) | — | — | — | — | — | — |
| Tetraglycerin monooleate (SY-Glyster FMO-3S (produced by Sakamoto Yakuhin Kogyo Co., Ltd.)) | 15.00 | 15.00 | 15.00 | 15.00 | 15.00 | 15.00 |
| Decaglycerin monolaurate (NIKKOL DECAGLYN 1-L (produced by Nikko Chemicals Co., Ltd.)) | 3.00 | 3.00 | 3.00 | 3.00 | 3.00 | 3.00 |
| Sucrose stearate (HLB19) (DK ESTER SS (produced by DKS Co. Ltd.)) | 6.00 | 6.00 | 6.00 | 6.00 | 6.00 | 6.00 |
| Sucrose stearate (HLB5) (RYOTO Sugar Ester S-570 (produced by Mitsubishi-Kagaku Foods Corp.)) | — | — | — | — | — | — |
| Sucrose laurate (HLB16) ("RYOTO Sugar Ester L-1695 (produced by Mitsubishi-Kagaku Foods Corp.)) | 3.80 | 3.80 | 3.80 | 3.80 | 3.80 | 3.80 |
| Sucrose laurate (HLB1) "RYOTO Sugar Ester L-195 (produced by Mitsubishi-Kagaku Foods Corp.)) | — | — | — | — | — | — |
| Purified water | 5.20 | 5.20 | 5.20 | 5.20 | 5.20 | 5.20 |
| Ethanol | 1.00 | — | — | — | — | — |
| Propylene glycol | — | 1.00 | — | — | — | — |
| Xylitol | — | — | 1.00 | — | — | — |
| Sorbitol | — | — | — | 1.00 | — | — |
| Lactitol | — | — | — | — | 1.00 | — |
| Erythritol | — | — | — | — | — | 1.00 |
| Glycerin | 9.00 | 9.00 | 9.00 | 9.00 | 9.00 | 9.00 |
| Total (g) | 100 | 100.0 | 100.0 | 100.0 | 100.0 | 100.0 |
| Homogeneity of formulation (state of dispersion) | O | O | O | O | O | O |

| | International application No. |
|---|---|
| | PCT/JP2019/009549 |

A. CLASSIFICATION OF SUBJECT MATTER
Int.Cl.  See extra sheet

According to International Patent Classification (IPC) or to both national classification and IPC

B. FIELDS SEARCHED

Minimum documentation searched (classification system followed by classification symbols)
Int.Cl.  A23L29/10,    A23L33/105,    A61K9/107,    A61K9/48,    A61K31/01,
          A61K31/015,    A61K31/045,    A61K31/047,    A61K31/122,    A61K36/05,
          A61K47/02, A61K47/10, A61K47/14, A61K47/26, A61P17/18, A61P27/02,
          A61P29/00, A61P35/00, A61P39/06

Documentation searched other than minimum documentation to the extent that such documents are included in the fields searched
       Published examined utility model applications of Japan        1922-1996
       Published unexamined utility model applications of Japan       1971-2019
       Registered utility model specifications of Japan               1996-2019
       Published registered utility model applications of Japan       1994-2019

Electronic data base consulted during the international search (name of data base and, where practicable, search terms used)
       JSTPlus/JMEDPlus/JST7580 (JDreamIII), CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 5094271 B2 (FUJIFILM CORP.) 12 December 2012, paragraphs [0001], [0049]-[0053], [0060]-[0061], example 1 & JP 2009-46143 A & US 2011/0104340 A1, paragraphs [0002], [0034]-[0045], [0064]-[0065], example 1 & WO 2009/025380 A2 & EP 2185010 A2 & CN 101778575 A & TW 200932127 A | 1-5<br>6-7 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 May 2019 (28.05.2019) | 11 June 2019 (11.06.2019) |

| Name and mailing address of the ISA/<br>    Japan Patent Office<br>    3-4-3, Kasumigaseki, Chiyoda-ku,<br>    Tokyo 100-8915, Japan | Authorized officer |
|---|---|
| | Telephone No. |

Form PCT/ISA/210 (second sheet) (January 2015)

## INTERNATIONAL SEARCH REPORT

International application No.

PCT/JP2019/009549

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2007-269749 A (FUJIFILM CORP.) 18 October 2007, paragraphs [0001], [0024]-[0033], example 2 (Family: none) | 1-5<br>6-7 |
| Y | JP 2012-206972 A (FUJIFILM CORP.) 25 October 2012, paragraphs [0035]-[0042], [0097] & US 2013/0172426 A1, paragraphs [0110]-[0115], [0229] & WO 2012/133005 A1 & EP 2606881 A1 & CN 103118673 A & KR 10-2013-0139869 A | 6-7 |
| P, X | JP 2018-177751 A (FUJIFILM CORP.) 15 November 2018, claims (Family: none) | 1-7 |

Form PCT/ISA/210 (continuation of second sheet) (January 2015)

| INTERNATIONAL SEARCH REPORT | International application No. |
|---|---|
| | PCT/JP2019/009549 |

CLASSIFICATION OF SUBJECT MATTER
A23L29/10(2016.01)i,    A23L33/105(2016.01)i,    A61K9/107(2006.01)i,
A61K9/48(2006.01)i,    A61K31/01(2006.01)i,    A61K31/015(2006.01)i,
A61K31/045(2006.01)i,    A61K31/047(2006.01)i,    A61K31/122(2006.01)i,
A61K36/05(2006.01)i,    A61K47/02(2006.01)i,    A61K47/10(2006.01)i,
A61K47/14(2006.01)i,    A61K47/26(2006.01)i,    A61P17/18(2006.01)i,
A61P27/02(2006.01)i,    A61P29/00(2006.01)i,    A61P35/00(2006.01)i,
A61P39/06(2006.01)i

**EP 3 760 053 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012206972 A
- WO 1999050384 A
- JP 8103288 A
- JP 5068585 A

### Non-patent literature cited in the description

- **TAKAHASHI, JIRO et al.** Toxicity Test of Haematococcus Algae Astaxanthin - Ames Test, Toxicity Test with Single Administration to Rats, Toxicity Test with Repetitive Oral Administration to Rats for 90 Days. *Journal of Clinical Therapeutics & Medicines,* 2004, vol. 20, 867-881
- **RENSTROM, B. et al.** Fatty acids of some esterified carotenols. *Comp. Biochem. Physiol. B, Comp. Biochem.,* 1981, vol. 69, 625-627
- **YAMAGUCHI, K. et al.** The composition of carotenoid pigments in the Antarctic krill Euphausia superba. *Bull. Jap. Sos. Sci. Fish.,* 1983, vol. 49, 1411-1415
- **ANDREWES, A. G. et al.** 3R,3'R)-Astaxanthin from the yeast Phaffia rhodozyma. *Phytochem.,* 1976, vol. 15, 1009-1011
- **ANDREWES, A. G. et al.** Carotenids of Phaffia rhodozyma, a red pigmented fermenting yeast. *Phytochem.,* 1976, vol. 15, 1003-1007
- The handbook of oil chemistry: lipids and surfactants. 2001
- **KAZIMOTO ; SANSHU SHOBO.** *Theory and Practical Use of Antioxidants,* 1984
- **SARUWATARI et al.** Handbook of Antioxidants. TAISEISHA LTD, 1976